# FASCICULE DE BREVET EUROPEEN

(11) **EP 1 044 970 B1**
(45) Date de publication et mention de la délivrance du brevet: **15.01.2003**
(21) Numéro de dépôt: 00401039.3
(22) Date de dépôt: 14.04.2000
(51) Int. Cl.: C07D 249/14, C07D 401/04, C07D 403/12, C07D 401/14, C07D 401/12, C07D 405/12, C07D 409/12, A61K 31/4196, A61P 3/00

(54) **Nouveaux composés aminotriazoles, leur procédé de préparation et les compositions pharmaceutiques qui les contiennent**
Aminotriazolverbindungen, Verfahren zu deren Herstellung und sie enthaltende pharmazeutische Zusammensetzungen
Aminotriazole compounds, process for their preparation and pharmaceutical compositions containing them

(30) Priorité: 15.04.1999 FR 9904721
(43) Date de publication de la demande: 18.10.2000
(73) Titulaire: LES LABORATOIRES SERVIER, 92200 Neuilly sur Seine (FR)
(72) Inventeur: Fauchere, Jean-Luc, 92210 Saint Cloud (FR); Ortuno, Jean-Claude, 78000 Versailles (FR); Duhault, Jacques, 44410 Saint Lyphard (FR); Boutin, Jean Albert, 92150 Suresnes (FR); Levens, Nigel, 92420 Vaucresson (FR)

(56) Documents cités:
- WO-A-94/17035
- WO-A-98/27063
- WO-A-99/32466

## Description

La présente invention concerne de nouveaux composés aminotriazoles, leur procédé de préparation et les compositions pharmaceutiques qui les contiennent.

Les composés de la présente invention possèdent une structure originale caractérisée par l'association d'un groupement aminotriazole, d'un motif hydrazide et d'un espaceur de type aromatique. Ces composés trouvent leur utilisation dans le traitement des pathologies liées au neuropeptide Y (NPY).

Le Neuropeptide Y (NPY) est un peptide de 36 acides aminés, proche du peptide YY (PYY) et des polypeptides pancréatiques (PP). A l'origine isolé du cerveau de porc (Proc. Natl. Acad. Sci., 1982, 79, 5485), le NPY est largement distribué chez les mammifères au niveau du système nerveux central et périphérique. Ce neurotransmetteur est présent en fortes concentrations dans les fibres nerveuses du cerveau, mais aussi du coeur, des ganglions sympathiques, des vaisseaux sanguins et des muscles lisses du vas deferens et du tractus gastrointestinal. Il est responsable d'effets physiologiques divers qui s'exercent par l'intermédiaire de récepteurs spécifiques (Y). Ces derniers forment un groupe hétérogène dont 6 sous-types ont été identifiés jusqu'à aujourd'hui : Y₁ à Y₆ (Pharmacological Reviews, 1998, 50, 143). Le NPY est impliqué dans le comportement alimentaire, en stimulant fortement la prise de nourriture (Proc. Natl. Acad. Sci., 1985, 82, 3940), ou en exerçant un rôle de régulateur sur l'axe HPA (Hypothalamique-Pituitaire-Surrénal) (J. of Neuroendocrinol., 1995, 7, 273). Il présente également des propriétés anxiolytiques et sédatives (Neuropsychopharmacology, 1993, 8, 357), un fort pouvoir vasoconstricteur (Eur. J. Pharmacol., 1984, 85, 519) qui induit une augmentation de la pression sanguine, et possède aussi un effet sur le rythme circadien (Neuroscience and Biobehavioral Reviews, 1995, 19, 349).

Différents ligands des récepteurs NPY ont été décrits récemment. A titre d'exemple on peut citer des composés peptidiques cycliques (WO 9400486), des aminoacides dérivés de l'arginine (WO 9417035) ou des composés non peptidiques (WO 9827063).

Les composés de l'invention, outre le fait qu'ils soient nouveaux, ont montré une action inhibitrice in vivo sur la prise de nourriture et la prise de poids. Cet effet s'exerce par l'intermédiaire d'une liaison avec les récepteurs du NPY. Les composés de l'invention pourront donc être utilisés dans le traitement des pathologies nécessitant un ligand des récepteurs du NPY, en particulier dans le traitement des pathologies liées à des troubles du comportement alimentaire ou de la balance énergétique, telles que le diabète, l'obésité, la boulimie, l'anorexie mentale, mais également dans le traitement de l'hypertension artérielle, de l'anxiété, des dépressions, de l'épilepsie, des dysfonctionnements sexuels et des troubles du sommeil.

La présente invention concerne particulièrement les composés de formule (I) : 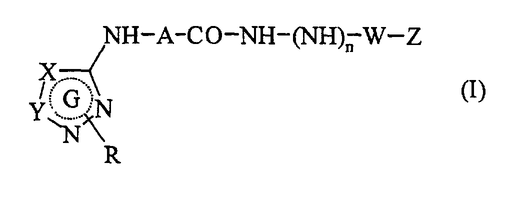 dans laquelle :
- n vaut 0 ou 1,
- W représente un groupement -CO- ou un groupement S(O)_{q} dans lequel q vaut 0. 1 ou 2,
- Le groupement 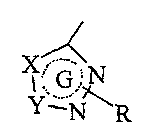 représente un groupement choisi parmi : 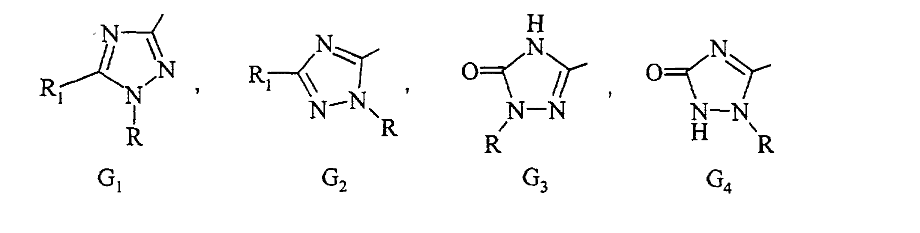
- Z représente un groupement alkyle, aryle éventuellement substitué, hétéroaryle éventuellement substitué, arylalkyle éventuellement substitué, arylalkényle éventuellement substitué, arylalkynyle éventuellement substitué, hétéroarylalkyle éventuellement substitué, hétéroarylalkényle éventuellement substitué ou hétéroarylalkynyle éventuellement substitué,
- A représente un groupement choisi parmi -A₂-, -A₁-A₂-, -A₂-A₁- ou -A₁-A₂-A₁- dans lesquels A₁ est un groupement alkylène et A₂ représente un groupement phénylène éventuellement substitué, naphtylène éventuellement substitué, cycloalkylène, ou hétéroarylène éventuellement substitué,
- R représente un atome d'hydrogène, un groupement alkyle, aryle éventuellement substitué, hétéroaryle éventuellement substitué, arylalkyle éventuellement substitué, arylalkényle éventuellement substitué, arylalkynyle éventuellement substitué, hétéroarylalkyle éventuellement substitué, hétéroarylalkényle éventuellement substitué, ou hétéroarylalkynyle éventuellement substitué,
- R₁ représente un groupement alkyle, aryle éventuellement substitué, hétéroaryle éventuellement substitué, arylalkyle éventuellement substitué, arylalkényle éventuellement substitué, arylalkynyle éventuellement substitué, hétéroarylalkyle éventuellement substitué, hétéroarylalkényle éventuellement substitué, ou hétéroarylalkynyle éventuellement substitué,
leurs énantiomères, diastéréoisomères, ainsi que leurs sels d'addition à un acide ou à une base pharmaceutiquement acceptable,
étant entendu que,
- le terme alkyle désigne un groupement linéaire ou ramifié de 1 à 6 atomes de carbone,
- le terme alkylène désigne un radical bivalent, linéaire ou ramifié, contenant de 1 à 6 atomes de carbone,
- le terme alkényle désigne un groupement linéaire ou ramifié contenant de 2 à 6 atomes de carbone et de 1 à 3 double liaison,
- le terme alkynyle désigne un groupement linéaire ou ramifié contenant de 2 à 6 atomes de carbone et de 1 à 3 triple liaison,
- le terme aryle désigne un groupement phényle, napthyle, biphényle, dihydronapthyle, ou tétrahydronapthyle,
- le terme hétéroaryle désigne un groupement mono ou bicyclique insaturé ou partiellement insaturé, de 5 à 11 chaînons, contenant de 1 à 4 hétéroatomes choisis parmi azote, oxygène et soufre.
- les termes phénylène et naphtylène désignent respectivement des radicaux phényle et naphtyle bivalents,
- le terme cycloalkylène désigne un groupement bivalent cyclique saturé contenant de 3 à 8 atomes de carbone,
- le terme hétéroarylène désigne un radical bivalent hétéroaryle tel que défini précédemment,
l'expression "éventuellement substitué" affectée aux termes aryle, arylalkyle, hétéroaryle, ou hétéroarylalkyle, signifie que ces groupements sont substitués sur leur partie cyclique par 1 à 5 substituants, identiques ou différents, choisis parmi alkyle (C₁-C₆) linéaire ou ramifié, alkoxy (C₁-C₆) linéaire ou ramifié, halogène, hydroxy, perhalogénoalkyle (C₁-C₆) linéaire ou ramifié, nitro, amino (éventuellement substitué par un ou deux groupements alkyle (C₁-C₆) linéaire ou ramifié), acyle (C₁-C₆) linéaire ou ramifié, aminocarbonyle (éventuellement substitué sur l'atome d'azote par un ou deux groupements alkyle (C₁-C₆) linéaire ou ramifié), acylamino (C₁-C₆) linéaire ou ramifié, alkoxycarbonyle (C₁-C₆) linéaire ou ramifié, formyle, carboxy, sulfo, nitrile, aminoalkyle (C₁-C₆) linéaire ou ramifié (éventuellement substitué sur l'atome d'azote par un ou deux groupements alkyle (C₁-C₆) linéaire ou ramifié), thioalkyle (C₁-C₆) linéaire ou ramifié (éventuellement substitué sur l'atome de soufre par un groupement alkyle (C₁-C₆) linéaire ou ramifié), ou hydroxyalkyle (éventuellement substitué sur l'atome d'oxygène par un groupement alkyle (C₁-C₆) linéaire ou ramifié),
l'expression "éventuellement substitué" affectée aux termes phénylène, naphtylène ou hétéroarylène signifie que ces groupements sont substitués par un à trois groupements identiques ou différents, choisis parmi alkyle (C₁-C₆) linéaire ou ramifié, alkoxy (C₁-C₆) linéaire ou ramifié, halogène, hydroxy, perhalogénoalkyle (C₁-C₆) linéaire ou ramifié, nitro, amino (éventuellement substitué par un ou deux groupements alkyle (C₁-C₆) linéaire ou ramifié), acyle (C₁-C₆) linéaire ou ramifié, formyle, carboxy, alkoxycarbonyle (C₁-C₆) linéaire ou ramifié, aminocarbonyle (éventuellement substitué sur l'atome d'azote par un ou deux groupements alkyle (C₁-C₆) linéaire ou ramifié), acylamino (C₁-C₆) linéaire ou ramifié, ou nitrile.

Parmi les groupements hétéroaryles on préférera les groupements pyridyle, furyle, thiényle, et indolyle.

Parmi les groupements hétéroarylènes on préférera les groupements pyridinylène, et pyrazinylène.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

Un aspect avantageux de l'invention concerne les composés de formule (I) pour lesquels n vaut 1.

Un autre aspect avantageux de l'invention concerne les composés de formule (I) pour lesquels n vaut 0.

Des composés préférés de l'invention sont ceux pour lesquels W représente un groupement SO₂.

Des composés préférés de l'invention sont ceux pour lesquels le groupement 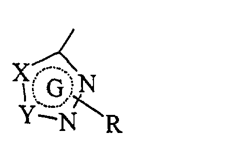 représente un groupement choisi parmi :

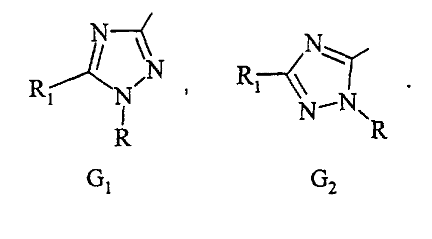

D'autres composés préférés de l'invention sont ceux pour lesquels le groupement 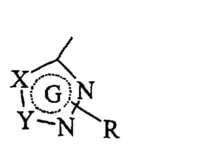 représente un groupement choisi parmi :

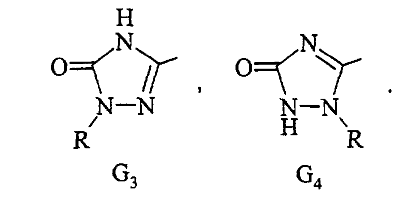

Dans des composés préférés de formule (I), A représente un groupement A₂, A₂ étant plus particulièrement un groupement phénylène, pyridinylène, ou pyrazinylène.

Dans d'autres composés préférés de formule (I), A représente un groupement -A₁-A₂- ou -A₂-A₁-, A₂ étant plus particulièrement un groupement phénylène, pyridinylène, ou pyrazinylène.

Dans les composés de formule (I), R₁ représente préférentiellement un groupement aryle éventuellement substitué.

Dans les composés de formule (I), R sera avantageusement choisi parmi hydrogène, et un groupement aryle éventuellement substitué, (plus particulièrement phényle) et un groupement hétéroaryle éventuellement substitué (plus particulièrement pyridinyle, furyle ou thiényle).

Un aspect avantageux de l'invention concerne les composés de formule (I) pour lesquels Z représente un groupement choisi parmi alkyle, aryle éventuellement substitué et hétéroaryle éventuellement substitué.

La présente invention concerne de façon particulièrement avantageuse les composés de formule (I) pour lesquels n vaut 1, W représente un groupement SO₂, A représente un groupement choisi parmi phénylène, pyridinylène, et pyrazinylène, R₁ représente un groupement aryle éventuellement substitué, R est choisi parmi un atome d'hydrogène, un groupement aryle éventuellement substitué et hétéroaryle éventuellement substitué, et Z représente un groupement alkyle, aryle éventuellement substitué ou hétéroaryle éventuellement substitué.

Le groupement aryle préféré de l'invention est le groupement phényle.

L'invention concerne tout particulièrement les composés suivants :
- N'-[4-({5-Phényl-1-[3-(trifluorométhyl)phényl]-*1H*-1,2,4-triazol-3-yl}amino)benzoyl] benzènesulfonohydrazide
- 4-Méthoxy-N'-[4-({5-phényl-1-[3-trifluorométhyl)phényl]-*1H*-1,2,4-triazol-3-yl} amino)benzoyl]benzènesulfonohydrazide
- N'-{4-[(5-Phényl-1*H-*1,2,4-triazol-3-yl)amino]benzoyl}benzènesulfonohydrazide
- N'-(4-{[5-Phényl-1-(2-pyridyl)-*1H*-1,2,4-triazol-3-yl]amino}benzoyl) benzènesulfonohydrazide
- N'-[4-({5-Oxo-1-[3-(trifluorométhyl)phényl]-4,5-dihydro-*1H*-1,2,4-triazol-3-yl}amino) benzoyl}benzènesulfonohydrazide
- N'-(4-{[5-Oxo-1-(2-pyridyl)-4,5-dihydro-*1H*-1,2,4-triazol-3-yl]amino}benzoyl) benzènesulfonohydrazide
- N'-[(6-{[5-oxo-1-(2-pyridinyl)-4,5-dihydro-1*H*-1,2,4-triazol-3-yl]amino}-3-pyridinyl) carbonyl]benzènesulfonohydrazide

La présente invention concerne également le procédé de préparation des composés de formule (I) caractérisé en ce que l'on utilise comme produit de départ un composé de formule (II) : 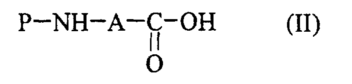 dans laquelle A a la même définition que dans la formule (I) et P représente un groupement protecteur de la fonction amine,
qui réagit en présence d'un agent de couplage avec un composé de formule (III) :

NH₂-(NH)ₙ-W-Z (III)

dans laquelle n, W et Z sont tels que définis dans la formule (I),
pour conduire, après déprotection de la fonction amine par clivage du groupement P, à un composé de formule (IV) : 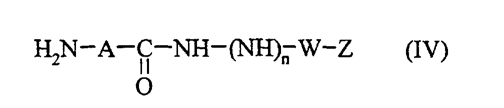 dans laquelle n, A, W et Z sont tels que définis précédemment,
composé (IV) qui est ensuite condensé en milieu basique sur un isothiocyanate de formule (V) : 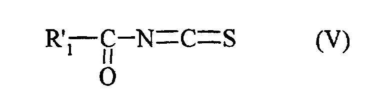 dans laquelle R'₁ a la même signification que R₁ dans la formule (I) ou bien représente un groupement alkoxy (C₁-C₆) linéaire ou ramifié,
pour conduire à un composé de formule (VI) : 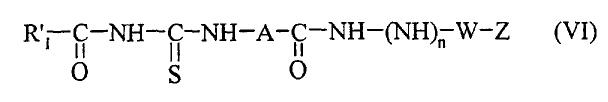 dans laquelle n, R'₁, A, W et Z sont tels que définis précédemment,
composé de formule (VI) qui est :
- soit, lorsque R'₁ représente un groupement alkoxy (C₁-C₆) linéaire ou ramifié, condensé en présence d'un agent de couplage sur une hydrazine de formule R-NH-NH₂, dans laquelle R est tel que défini dans la formule (I), pour conduire à un composé de formule (VII/a) : 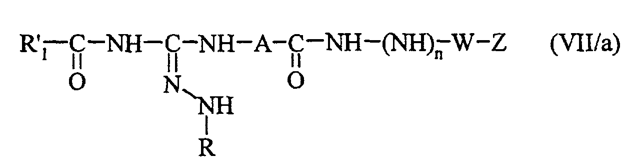
dans laquelle R, A, n, W et Z sont tels que définis précédemment, et R'₁ représente un groupement alkoxy (C₁-C₆) linéaire ou ramifié,
composé (VII/a) qui se cyclise, spontanément ou après traitement en milieu acide, suivant la nature du groupement R, pour conduire au mélange des deux composés de formule (I/a) et (I/b) : 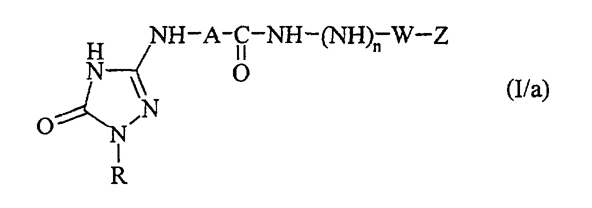 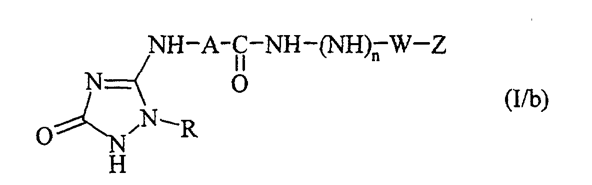 cas particuliers des composés de formule (I) pour lesquels R, A, n, W et Z sont tels que définis précédemment,
composés (I/a) et (I/b) qui peuvent être séparés selon des techniques classiques de séparation,
- soit, lorsque R'₁ représente un groupement R₁ tel que défini dans la formule (I), condensé en présence d'un agent de couplage sur une hydrazine de formule R-NH-NH₂, dans laquelle R est tel que défini dans la formule (I), pour conduire à un composé de formule (VII/b) : 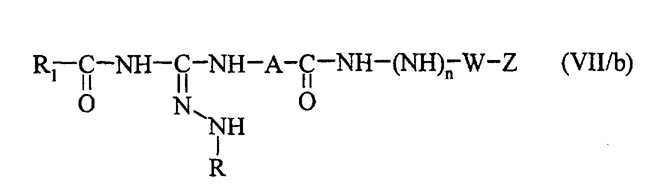
dans laquelle R₁, R, A, n, W et Z sont tels que définis précédemment,
composé (VII/b) qui subit une réaction de cyclisation suivie d'une deshydration, de façon spontanée ou après traitement en milieu acide, suivant la nature du groupement R, pour conduire au mélange des deux composés de formule (I/c) et (I/d) : 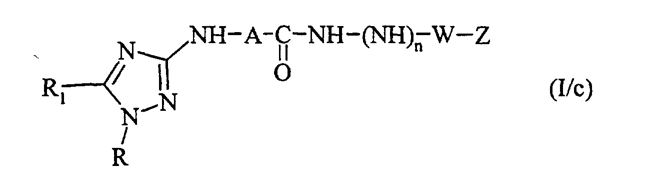 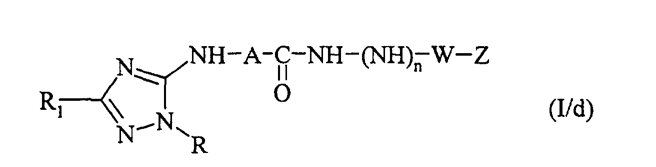 cas particuliers des composés de formule (I) pour lesquels R₁, R, A, n, W et Z sont tels que définis précédemment,
composés (I/c) et (I/d) qui peuvent être séparés selon des techniques classiques de séparation,
les composés (I/a), (I/b), (I/c) et (I/d) formant la totalité des composés de formule (I),
- dont on sépare, le cas échéant, les énantiomères et/ou diastéréoisomères selon une technique classique de séparation,
- que l'on transforme, si on le souhaite, en leurs sels d'addition à un acide ou à une base pharmaceutiquement acceptable.

La présente invention a également pour objet les compositions pharmaceutiques renfermant comme principe actif au moins un composé de formule (I), seul ou en combinaison avec un ou plusieurs excipients ou véhicules inertes non toxiques, pharmaceutiquement acceptable.

Parmi les compositions pharmaceutiques selon l'invention, on pourra citer plus particulièrement celles qui conviennent pour l'administration orale, parentérale, nasale, transdermique, les comprimés simples ou dragéifiés, les comprimés sublinguaux, les gélules, les tablettes, les suppositoires, les crèmes, pommades, gels dermiques, etc...

La posologie utile varie selon l'âge et le poids du patient, la nature et la sévérité de l'affection ainsi que la voie d'administration. Celle-ci peut être orale, nasale, rectale ou parentérale. D'une manière générale, la posologie unitaire s'échelonne entre 0,05 et 500 mg pour un traitement en 1 à 3 prises par 24 heures.

Les exemples suivants illustrent l'invention et ne la limitent en aucune façon. Les structures des composés décrits ont été confirmées par les techniques spectroscopiques usuelles.

Les produits de départ utilisés sont des produits connus ou préparés selon des modes opératoires connus.

### EXEMPLE 1 : N'-[4-({5-Phényl-1-[3-(trifluorométhyl)phényl]-1H-1,2,4-triazol-3-yl} amino)benzoyl]benzènesulfonohydrazide

### Stade a : 4-{[2-(Phénylsulfonyl)hydrazino]carbonyl}phénylcarbamate de tert-butyle

A une solution de 12 mmol (2,85 g) d'acide 4-[(tert-butoxycarbonyl)amino]benzoïque dans 20 ml de diméthylformamide sont ajoutées successivement 12,1 mmol (1,65 g) de 1-hydroxy-7-azabenzotriazole et 18 mmol (3,45 g) d'EDCI. La réaction est agitée à température ambiante pendant une heure, et 18 mmol (3,1 g) de benzènesulfonohydrazide sont ajoutées. Après huit heures d'agitation à température ambiante le milieu réactionnel est versé dans 100 ml d'une solution d'acide chlorhydrique à 10 % et extrait quatre fois par 50 ml d'acétate d'éthyle. La phase organique est lavée deux fois avec 50 ml d'eau puis trois fois par une solution aqueuse saturée de bicarbonate de sodium et une fois par 50 ml d'une solution aqueuse saturée de chlorure de sodium. Après séchage sur sulfate de magnésium, filtration et concentration, le produit attendu est obtenu.

### Stade b : N'-(4-(Aminobenzoyl)benzènesulfonohydrazide, chlorhydrate

Le composé décrit au stade précédent est dissout dans 40 ml d'acide chlorhydrique 4M dans le dioxane. Après une nuit d'agitation à température ambiante, le solvant est évaporé à température ambiante. Le résidu est mis en suspension dans 300 ml d'éther, et filtré. Le solide obtenu est lavé 4 fois par 30 ml d'éther puis séché sous pression réduite, pour conduire au produit attendu.

### Stade c : N- Benzoyl-N'-(4-{[2-phénylsulfonyl)hydrazino]carbonyl}phényl)thiourée

A une suspension de 11,1 mmol (3,65 g) du composé décrit au stade précédent dans 20 ml d'acétonitrile sont ajoutées 11,1 mmol (0,66 ml) de diisopropyléthylamine. Après solubilisation, 13,9 mmol (1,86 ml) de isothiocyanate de benzoyle sont ajoutées. Le milieu réactionnel est agité 8 heures à température ambiante. Le précipité formé est filtré, lavé deux fois par 5 ml d'acétonitrile et 4 fois par 25 ml d'éther, pour conduire après séchage au produit attendu.

### Stade d : N'-[4-({5-Phényl-1-[3-(trifluorométhyl)phényl]-1H-1,2,4-triazol-3-yl} amino)benzoyl]benzènesulfonohydrazide

A une solution de 3,08 mmol (1,4 g) du composé décrit au stade précédent dans 5 ml de diméthylformamide sont ajoutées 3,4 mmol (0,528 ml) de 3(trifluorométhyl)phénylhydrazine et 6,16 mmol (1,41 g) d'EDCI. Le milieu réactionnel est agité à température ambiante pendant une nuit. Le milieu est versé dans 10 ml de HC1 10 % aqueux puis extrait 3 fois avec 100 ml d'acétate d'éthyle. Les phases organiques sont rassemblées et lavées 1 fois avec 20 ml d'eau, 2 fois avec 20 ml d'une solution aqueuse saturée en bicarbonate de sodium et 1 fois avec une solution aqueuse saturée en chlorure de sodium. La phase organique est séchée sur sulfate de magnésium filtrée et évaporée.
Le produit obtenu est purifié par chromatographie sur gel de silice en utilisant comme éluant un mélange dichlorométhane/acétate d'éthyle 75/25, pour conduire au produit titre.
*Spectre de masse :* ESI-MS : MH⁺ = 579

Les composés des exemples 2 à 25 sont obtenus selon le procédé décrit dans l'exemple 1, en utilisant les sulfonohydrazides, isothiocyanates et hydrazines appropriés aux stades a, c et d respectivement.

### EXEMPLE 2 : N'-[4-({5-[2-Chlorophényl]-1-[3-(trifluorométhyl)phényl]-1H-1,2,4-triazol-3-yl}amino)benzoyl]benzènesulfonohydrazide

*Spectre de masse :* ESI-MS : MH⁺ = 613

### EXEMPLE 3 : N'-[4-({5-[2-Chlorophényl]-1-phényl-1H-1,2,4-triazol-3-yl}amino) benzoyl]benzènesulfonohydrazide

*Spectre de masse :* ESI-MS : MH⁺ = 545

### EXEMPLE 4 : N'-{4-[(1,5-Diphényl-1H-1,2,4-triazol-3-yl)amino]benzoyl}-4-méthoxy benzènesulfonohydrazide

### EXEMPLE 5 : N'-(4-{[1-{4-Fluorophényl)-5-phényl-1H-1,2,4-triazol-3-yl]amino} benzoyl)-4-méthoxybenzènesulfonohydrazide

### EXEMPLE 6 : 4-Méthoxy-N'-[4-({5-phény]-1-[3-trifluorométhyl)phényl]-1H-1,2,4-triazol-3-yl}amino)benzoyl]benzènesulfonohydrazide

*Spectre de masse :* ESI-MS : MH⁺ = 609

### EXEMPLE 7 : N'-(4-{[1-(4-Fluorophényl)-5-phényl-1H-1,2,4-triazol-3-yl]amino} benzoyl)-4-méthoxybenzènesulfonohydrazide

### EXEMPLE 8 : N'-{4-[(1,5-Diphényl-1H-1,2,4-triazol-3-yl)amino]benzoyl} benzènesulfonohydrazide

### EXEMPLE 9 : N'-[4-({5-[4-Chlorophényl]-1-[3-(trifluorométhyl)phényl]-1H-1,2,4-triazol-3-yl}amino)benzoyl]benzènesulfonohydrazide

### EXEMPLE 10 : N'-(4-{[5-(4-Chlorophényl)-1-phényl-1H-1,2,4-triazol-3-yl]amino} benzoyl)benzènesulfonohydrazide

*Spectre de masse :* ESI-MS : MH⁺ = 546

### EXEMPLE 11 : N'-(4-{[5-(4-Chlorophényl)-1-(4-fluorophényl)-1H-1,2,4-triazol-3-yl] amino}benzoyl)benzènesulfonohydrazide

*Spectre de masse :* ESI-MS : MH⁺ = 564

### EXEMPLE 12 : N'-[4-({5-[4-Méthoxyphényl]-1-[3-(trifluorométhyl)phényl]-1H-1,2,4-triazol-3-yl}amino)benzoyl]benzènesulfonohydrazide

*Spectre de masse :* ESI-MS : MH⁺ = 609

### EXEMPLE 13 : N'-(4-{[5-(4-Méthoxyphényl)-1-phényl-1H-1,2,4-triazol-3-yl]amino} benzoyl)benzènesulfonohydrazide

*Spectre de masse :* ESI-MS : MH⁺ = 541

### EXEMPLE 14 : N'-(4-{[1-(4-Fluorophényl)-5-(4-méthoxyphényl)-1H-1,2,4-triazol-3-yl]amino}benzoyl)benzènesulfonohydrazide

*Spectre de masse :* ESI-MS : MH⁺ = 559

### EXEMPLE 15 : N'-{4-[(1-Benzyl-5-phényl-1H-1,2,4-triazol-3-yl)amino]benzoyl} benzènesulfonohydrazide

*Spectre de masse :* ESI-MS : MH⁺ = 525

### EXEMPLE 16 : N'-{4-[(5-Phényl-1H-1,2,4-triazol-3-yl)amino]benzoyl}benzène sulfonohydrazide

*Spectre de masse :* ESI-MS : MH⁺ = 435

### EXEMPLE 17 : N'-(4-{[5-Phényl-1-(2-pyridyl)-1H-1,2,4-triazol-3-yl]amino}benzoyl) benzènesulfonohydrazide

*Spectre de masse :* ESI-MS : MH⁺ = 512

### EXEMPLE 18 : N'-[4-({5-Phényl-1-[4-(trifluorométhyl)phényl]-1H-1,2,4-triazol-3-yl}amino)benzoyl]benzènesulfonohydrazide

*Spectre de masse :* ESI-MS : MH⁺ = 579

### EXEMPLE 19 : N'-[4-({5-Phényl-1-[2-(trifluorométhyl)phény]-1H-1,2,4-triazol-3-yl}amino)benzoyl]benzènesulfonohydrazide

### EXEMPLE 20: 4-Méthyl-N'-[4-({5-phényl-1-[3-(trifluorométhyl)phényl]-1H-1,2,4-triazol-3-yl}amino)benzoyl]benzènesulfonohydrazide

*Spectre de masse :* ESI-MS : MH⁺ = 593

### EXEMPLE 21 : N'-[4-({5-Phényl-1-[3-(trifluorométhyl)phényl]-1H-1,2,4-triazol-3-yl}amino)benzoyl]méthanesulfonohydrazide

*Spectre de masse :* ESI-MS MH⁺ = 517

### EXEMPLE 22 : 2,4,6-Trichloro-N'-[4-({5-phényl-1-[3-(trifluorométhyl)phényl]-1H-1,2,4-triazol-3-yl}amino)benzoyl]benzènesulfonohydrazide

### EXEMPLE 23 : 2,4,6-Triméthyl-N'-[4-({5-phényl-1-[3-(trifluorométhyl)phényl]-1H-1,2,4-triazol-3-yl}amino)benzoyl]benzènesulfonohydrazide

*Spectre de masse :* ESI-MS : MH⁺ = 621

### EXEMPLE 24 : 5-(Diméthylamino)-N'-[4-({5-phényl-1-[3-(trifluorométhyl)phényl]-1H-1,2,4-triazol-3-yl}amino)benzoyl]-1-naphtalènesulfonohydrazide

*Spectre de masse :* ESI-MS : MH⁺ = 672

### EXEMPLE 25 : N'-{4-[(1-Benzyl-5-phényl-1H-1,2,4-triazol-3-yl)amino]benzoyl}-4-méthoxysulfonohydrazide

*Spectre de masse :* ESI-MS : MH⁺ = 555

### EXEMPLE 26 : N-[4-({5-Phényl-1-[3-(trifluorométhyl)phényl]-1H-1,2,4-triazol-3-yl}amino)benzoyl]benzènesulfonamide

Le produit attendu est obtenu en utilisant le procédé décrit dans l'exemple 1, et en remplaçant, au stade a, le benzènesulfonohydrazide par le benzènesulfonamide.
*Spectre de masse :* ESI-MS : MH⁺ = 564

De la même façon que pour l'exemple 26, et en utilisant l'isothiocyanate et l'hydrazine appropriés aux stades c et d respectivement, les composés des exemples 27 et 28 sont obtenus.

### EXEMPLE 27 : N-{4-[(1,5-Diphényl-1H-1,2,4-triazol-3-yl)amino]benzoyl} benzènesulfonamide

*Spectre de masse :* ESI-MS : MH⁺ = 496

### EXEMPLE 28 : N-(4-{[1-(4-Fluorophényl)-5-phényl-1H-1,2,4-triazol-3-yl]amino} benzoyl)benzènesulfonamide

### EXEMPLE 29 : N'-[4-({5-Oxo-1-[3-(trifluorométhyl)phényl]-4,5-dihydro-1H-1,2,4-triazol-3-yl}amino}benzoyl}benzènesulfonohydrazide

### Stade a : (4-{[2-(Phénylsulfonyl)hydrazino]carbonyl}anilino)carbothioylcarbamate d'éthyle

Le produit attendu est obtenu selon le procédé décrit dans l'exemple 1, stade c, en remplaçant l'isothiocyanate de benzoyle par le thioxocarbamate d'éthyle.

### Stade b : N'-[4-({5-Oxo-1-[3-(trifluorométhyl)phényl]-4,5-dihydro-1H-1,2,4-triazol-3-yl}amino)benzoyl}benzènesulfonohydrazide

A une solution de 0,71 mmol (0,3 g) du composé décrit au stade précédent dans 3 ml de diméthylformamide sont ajoutées 1,065 mmol (0,139 ml) de 3(trifluorométhyl)phénylhydrazine et 1,42 mmol (0,272 g) d'EDCI. Le milieu réactionnel est agité à température ambiante pendant une nuit. Le milieu est versé dans 5 ml de HCl 10 % aqueux puis extrait 3 fois avec 5 ml d'acétate d'éthyle. Les phases organiques sont rassemblées et lavées 1 fois avec 5 ml d'eau. La phase organique est séchée sur sulfate de magnésium filtrée et évaporée. Le résidu est repris dans une solution à 10 % d'acide trifluoroacétique dans la dioxane et porté à 50°C pendant une nuit. Le milieu réactionnel est évaporé est le précipité formé au cours de l'évaporation est lavé par 2 ml d'acétonitrile et 2 fois 5 ml d'éther puis séché sous vide pour conduire au produit titre.
*Spectre de masse :* ESI-MS : MH⁺ = 519

Les composés des exemples 30 à 39 sont obtenus le procédé décrit dans l'exemple 29, en utilisant les sulfonohydrazides et hydrazines appropriées.

### EXEMPLE 30 : N'-{4-[(5-Oxo-1-phényl-4,5-dihydro-1H-1,2,4-triazol-3-yl)amino] benzoyl}benzènesulfonohydrazide

*Spectre de masse :* ESI-MS : MH⁺ = 451

### EXEMPLE 31 : N'-(4-{[1-(4-Fluorophényl)-5-oxo-4,5-dihydro-1H-1,2,4-triazol-3-yl]amino}benzoyl)benzènesulfonohydrazide

*Spectre de masse :* ESI-MS : MH⁺ = 469

### EXEMPLE 32 : N'-(4-{[5-Oxo-1-(2-pyridyl)-4,5-dihydro-1H-1,2,4-triazol-3-yl]amino} benzoyl)benzènesulfonohydrazide

*Spectre de masse :* ESI-MS : MH⁺ = 452

### EXEMPLE 33 : N'-{4-[(1-Benzyl-5-oxo-4,5-dihydro-1H-1,2,4-triazol-3-yl)amino] benzoyl}benzènesulfonohydrazide

### EXEMPLE 34 : 4-Méthyl-N'-[4-({5-oxo-1-[3-(trifluorométhyl)phényl]-4,5-dihydro-1H-1,2,4-triazol-3-yl}amino)benzoyl]benzènesulfonohydrazide

*Spectre de masse :* ESI-MS : MH⁺ = 533

### EXEMPLE 35 : 4-Méthoxy-N'-[4-({5-oxo-1-[3-(trifluorométhyl)phényl]-4,5-dihydro-1H-1,2,4-triazol-3-yl}amino}benzoyl]benzènesulfonohydrazide

*Spectre de masse :* ESI-MS : MH⁺ = 549

### EXEMPLE 36 : 4-Méthoxy-N'-{4-[(5-oxo-1-phényl-4,5-dihydro-1H-1,2,4-triazol-3-yl) amino]benzoyl}benzènesulfonohydrazide

### EXEMPLE 37 : N'-(4-{[1-(4-Fluorophényl)-5-oxo-4,5-dihydro-1H-1,2,4-triazol-3-yl] amino}benzoyl)-4-méthoxybenzènesulfonohydrazide

### EXEMPLE 38 : N'-{4-[(1-Benzyl-5-oxo-4,5-dihydro-1H-1,2,4-triazol-3-yl}amino] benzoyl}-4-méthoxybenzènesulfonohydrazide

### EXEMPLE 39 : N'-Benzoyl-4-({5-phényl-1-[3-(trifluorométhyl)phényl]-1H-1,2,4-triazol-3-yl)amino)benzohydrazide

Le produit attendu est obtenu selon le procédé décrit dans l'exemple 1, en remplaçant au stade a, le benzènesulfonohydrazide par le benzohydrazide.
*Spectre de masse :* ESI-MS : MH⁺ = 543

De la même façon que dans l'exemple 39, en remplaçant le sulfonohydrazide par l'hydrazide correspondant et en utilisant les isothiocyanates et hydrazines appropriées, les composés des exemples 40 à 45 sont obtenus.

### EXEMPLE 40 : N'-Benzoyl-4-[(1,5-diphényl-1H-1,2,4-triazol-3-yl)amino] benzohydrazide

*Spectre de masse :* ESI-MS : MH⁺ = 475

### EXEMPLE 41 : N'-Benzoyl-4-{[1-(4-fluorophényl)-5-phényl-1H-1,2,4-triazol-3-yl] amino}benzohydrazide

*Spectre de masse :* ESI-MS : MH⁺ = 493

### EXEMPLE 42 : N'-Benzoyl-4-{[5-phényl-1-(2-pyridyl)-1H-1,2,4-triazol-3-yl]amino} benzohydrazide

*Spectre de masse :* ESI-MS : MH⁺ = 476

### EXEMPLE 43 : N'-Benzoyl-4-({1-[3,5-bis(trifluorométhyl)phényl]-5-phényl-1H-1,2,4-triazol-3-yl}amino)benzohydrazide

*Spectre de masse :* ESI-MS : MH⁺ = 611

### EXEMPLE 44 : 4-[(1,5-Diphényl-1H-1,2,4-triazol-3-yl)amino]-N'-(1-naphtoyl) benzohydrazide

*Spectre de masse :* ESI-MS : MH⁺ = 525

### EXEMPLE 45: 4-{[1-(4-Fluorophényl)-5-phényl-1H-1,2,4-triazol-3-yl]amino}-N'-(1-naphtoyl)benzohydrazide

*Spectre de masse :* ESI-MS : MH⁺ = 543

Les composés des exemples 46 à 48 sont obtenus selon le procédé décrit dans l'exemple 1 en utilisant les sulfonohydrazides, les isothiocyanates et les hydrazines appropriés aux stades a, c et d respectivement.

### EXEMPLE 46: 4-Méthyl-N'-{4-[(5-phényl)-1H-1,2,4-triazol-3-yl)amino]benzoyl} benzènesulfonohydrazide

*Spectre de masse :* ESI-MS : MH⁺ = 449

### EXEMPLE 47 : N'-{4-[(5-Phényl)-1H-1,2,4-triazol-3-yl)amino]benzoyl} méthanesulfonohydrazide

*Spectre de masse :* ESI-MS : MH⁺ = 373

### EXEMPLE 48 : 4-Méthoxy-N'-{4-[(5-phényl-1H-1,2,4-triazol-3-yl)amino]benzoyl} benzènesulfonohydrazide

Les composés des exemples 49 à 60 sont obtenus selon le procédé décrit dans l'exemple 1 en utilisant les sulfonohydrazides, les isothiocyanates et les hydrazines appropriés aux stades a, c et d respectivement, et en remplaçant au stade a l'acide 4-[(tert-butoxycarbonyl)amino]benzoïque par l'acide 3-[(tert-butoxycarbonyl)amino]benzoïque.

### EXEMPLE 49 : N'-[3-({5-Phényl-1-[3-(trifluorométhyl)phényl]-1H-1,2,4-triazol-3-yl} amino)benzoyl]benzènesulfonohydrazide

*Spectre de masse :* ESI-MS : MH⁺ = 579

### EXEMPLE 50 : N'-{3-[(1,5-Diphényl-1H-1,2,4-triazol-3-yl)amino]benzoyl} benzènesulfonohydrazide

*Spectre de masse :* ESI-MS : MH⁺ = 511

### EXEMPLE 51 : N'-(3-{[5-Phényl-1-(2-pyridinyl)-1H-1,2,4-triazol-3-yl]amino} benzoyl)benzènesulfonohydrazide

*Spectre de masse :* ESI-MS : MH⁺ = 512

### EXEMPLE 52 : N'-(3-{[5-(2-Chlorophényl)-1-phényl-1H-1,2,4-triazol-3-yl]amino} benzoyl)benzènesulfonohydrazide

*Spectre de masse :* ESI-MS : MH⁺ = 546

### EXEMPLE 53 : N'-(3-{[5-(2-Chlorophényl)-1-(2-pyridinyl)-1H-1,2,4-triazol-3-yl] amino}benzoyl)benzènesulfonohydrazide

*Spectre de masse :* ESI-MS : MH⁺ = 547

### EXEMPLE 54 : 4-Méthoxy-N'-[3-({5-phényl-1-[3-(trifluorométhyl)phényl]-1H-1,2,4-triazol-3-yl}amino)benzoyl]benzènesulfonohydrazide

*Spectre de masse :* ESI-MS : MH⁺ = 609

### EXEMPLE 55 : N'-{3-[(1,5-Diphényl-1H-1,2,4-triazol-3-yl)amino]benzoyl}-4-méthoxybenzènesulfonohydrazide

*Spectre de masse :* ESI-MS : MH⁺ = 541

### EXEMPLE 56: 4-Méthoxy-N'-(3-{[5-phényl-1-(2-pyridinyl)-1H-1,2,4-triazol-3-yl] amino}benzoyl)benzènesulfonohydrazide

*Spectre de masse :* ESI-MS : MH⁺ = 542

### EXEMPLE 57 : N'-[3-({5-(2-Chlorophényl)-1-[3-(trfluorométhyl)phényl]1H-1,2,4-triazol-3-yl}amino)benzoyl]-4-methoxybenzènesulfonohydrazide

*Spectre de masse :* ESI-MS : MH⁺ = 644

### EXEMPLE 58 : N'-(3-{[5-(2-Chlorophényl)-1-phényl-1H-1,2,4-triazol-3-yl]amino} benzoyl)-4-methoxybenzènesulfonohydrazide

*Spectre de masse :* ESI-MS : MH⁺ = 576

### EXEMPLE 59 : N'-(3-{[5-(2-Chlorophényl)-1-(2-pyridinyl)-1H-1,2,4-triazol-3-yl] amino}benzoyl)-4-methoxybenzènesulfonohydrazide

*Spectre de masse :* ESI-MS : MH⁺ = 577

### EXEMPLE 60 : N'-[3-({5-(2-Chlorophényl)-1-[3-(trifluorométhyl)phényl]-1H-1,2,4-triazol-3-yl}amino)benzoyl]benzènesulfonohydrazide

*Spectre de masse :* ESI-MS : MH⁺ = 614

Les composés des exemples 61 à 63 sont obtenus selon le procédé décrit dans l'exemple 1 en utilisant les sulfonohydrazides, les isothiocyanates et les hydrazines appropriés aux stades a, c et d respectivement, et en remplaçant au stade a l'acide 4-[(tert-butoxycarbonyl)amino]benzoïque par l'acide 4-{[(tert-butoxycarbonyl)amino] méthyl}benzoïque.

### EXEMPLE 61 : 4-Méthoxy-N'-{4-[({5-phényl-1-[3-(trifluorométhyl)phényl]-1H-1,2,4-triazol-3-yl}amino)méthyl]benzoyl}benzènesulfonohydrazide

*Spectre de masse :* ESI-MS : MH⁺ = 737

### EXEMPLE 62 : N'-(4-{[(1,5-Diphényl-1H-1,2,4-triazol-3-yl)amino]méthyl}benzoyl) benzènesulfonohydrazide

*Spectre de masse :* ESI-MS : MH⁺ = 639

### EXEMPLE 63 : N'-[4-({[5-Phényl-1-(2-pyridinyl)-1H-1,2,4-triazol-3-yl]amino} méthyl)benzoyl]benzènesulfonohydrazide

*Spectre de masse :* ESI-MS : MH⁺ = 754

Les composés des exemples 64 à 66 sont obtenus selon le procédé décrit dans l'exemple 1 en utilisant les sulfonohydrazides, les isothiocyanates et les hydrazines appropriés aux stades a, c et d respectivement, et en remplaçant au stade a l'acide 4-[(tert-butoxycarbonyl)amino]benzoïque par l'acide {4-[(tert-butoxycarbonyl)amino] phényl}acétique.

### EXEMPLE 64 : 4-Méthoxy-N'-{[4-({5-phényl-1-[3-(trifluorométhyl)phényl]-1H-1,2,4-triazol-3-yl}amino)phényl]acétyl}benzènesulfonohydrazide

*Spectre de masse :* ESI-MS : MH⁺ = 623

### EXEMPLE 65 : N'-({4-[(1,5-Diphényl-1H-1,2,4-triazol-3-yl)amino]phényl)acétyl)-4-méthoxybenzènesulfonohydrazide

*Spectre de masse :* ESI-MS : MH⁺ = 555

### EXEMPLE 66: 4-Méthoxy-N'-[(4-{[5-phényl-1-(2-pyridinyl)-1H-1,2,4-triazol-3-yl] amino}phényl)acétyl]benzènesulfonohydrazide

*Spectre de masse :* ESI-MS : MH⁺ = 556

Les composés des exemples 67 à 72 sont obtenus selon le procédé décrit dans l'exemple 1 en utilisant les sulfonohydrazides, les isothiocyanates et les hydrazines appropriés aux stades a, c et d respectivement, et en remplaçant au stade a l'acide 4-[(tert-butoxycarbonyl)amino]benzoïque par l'acide 6-[(tert-butoxycarbonyl)amino]-3-pyridazinecarboxylique.

### EXEMPLE 67 : 4-Méthoxy-N'-{[6-({5-phényl-1-[3-(trifluorométhyl)phényl]-1H-1,2,4-triazol-3-yl}amino)-3-pyridazinyl]carbonyl}benzènesulfonohydrazide

*Spectre de masse :* ESI-MS : MH⁺ = 611

### EXEMPLE 68 : N'-({6-[(1,5-Diphényl-1H-1,2,4-triazol-3-yl)amino]-3-pyridazinyl} carbonyl)-4-méthoxybenzènesulfonohydrazide

*Spectre de masse :* ESI-MS : MH⁺ = 543

### EXEMPLE 69 : 4-Méthoxy-N'-[(6-{[5-phényl-1-(2-pyridinyl)-1H-1,2,4-triazol-3-yl] amino}-3-pyridazinyl)carbonyl]benzènesulfonohydrazide

*Spectre de masse :* ESI-MS : MH⁺ = 544

### EXEMPLE 70 : N'-{[6-({5-Phényl-1-[3-(trifluorométhyl)phényl]-1H-1,2,4-triazol-3-yl}amino)-3-pyridazinyl]carbonyl}benzènesulfonohydrazide

*Spectre de masse :* ESI-MS : MH⁺ = 581

### EXEMPLE 71 : N'-({6-[(1,5-Diphényl-1H-1,2,4-triazol-3-yl)amino]-3-pyridazinyl} carbonyl)benzènesulfonohydrazide

*Spectre de masse :* ESI-MS : MH⁺ = 513

### EXEMPLE 72 : N'-[(6-{[5-Phényl-1-(2-pyridinyl)-1H-1,2,4-triazol-3-yl]amino}-3-pyridazinyl)carbonyl]benzènesulfonohydrazide

*Spectre de masse :* ESI-MS : MH⁺ = 514

Les composés des exemples 73 à 75 sont obtenus selon le procédé décrit dans l'exemple 1 en utilisant les sulfonohydrazides, les isothiocyanates et les hydrazines appropriés aux stades a, c et d respectivement, et en remplaçant au stade a l'acide 4-[(tert-butoxycarbonyl)amino]benzoïque par l'acide 6-[(tert-butoxycarbonyl)amino] nicotinique.

### EXEMPLE 73 : N'-{[6-({5-Phényl-1-[3-(trifluorométhyl)phényl]-1H-1,2,4-triazol-3-yl}amino)-3-pyridinyl]carbonyl}benzènesulfonohydrazide

*Spectre de masse :* ESI-MS : MH⁺ = 580

### EXEMPLE 74 : N'-({6-[(1,6-Diphényl-1H-1,2,4-triazol-3-yl)amino]-3-pyridinyl} carbonyl)benzènesulfonohydrazide

*Spectre de masse :* ESI-MS : MH⁺ = 512

### EXEMPLE 75 : N'-[(6-{[5-Phényl-1-(2-pyridinyl)-1H-1,2,4-triazol-3-yl]amino}-3-pyridinyl)carbonyl]benzènesulfonohydrazide

*Spectre de masse :* ESI-MS : MH⁺ = 513

Les composés des exemples 76 à 78 sont obtenus selon le procédé décrit dans l'exemple 1 en utilisant les sulfonohydrazides et les hydrazines appropriés.

### EXEMPLE 76 : 4-Méthyl-N'-[4-({5-oxo-1-[3-(trifluorométhyl)phényl]-4,5-dihydro-1H-1,2,4-triazol-3-yl}amino)benzoyl]benzènesulfonohydrazide

*Spectre de masse :* ESI-MS : MH⁺ = 533

### EXEMPLE 77: 2,4,6-Triméthyl-N'-[4-({5-oxo-1-[3-(trifluorométhyl)phényl]-4,5-dihydro-1H-1,2,4-triazol-3-yl}amino)benzoyl] benzènesulfonohydrazide

*Spectre de masse :* ESI-MS : MH⁺ = 561

### EXEMPLE 78 : N'-[4-({5-Oxo-1-[3-(trifluorométhyl)phényl]-4,5-dihydro-1H-1,2,4-triazol-3-yl}amino)benzoyl]méthanesulfonohydrazide

*Spectre de masse :* ESI-MS : MH⁺ = 457

### EXEMPLE 79 : N'-[(6-{[5-oxo-1-(2-pyridinyl)-4,5-dihydro-1H-1,2,4-triazol-3-yl] amino}-3-pyridinyl)carbonyl]benzènesulfonohydrazide

Le produit attendu est obtenu selon le procédé décrit dans l'exemple 1, stades a, b et c en remplaçant au stade a l'acide 4-[(tert-butoxycarbonyl)amino]benzoïque par l'acide 6-[(tert-butoxycarbonyl)amino]nicotinique, au stade c l'isothiocyanate de benzoyle par le thioxocarbamate d'éthyle, et selon le procédé décrit dans l'exemple 21 stade b en remplaçant la 3-(trifluorométhyl)phénylhydrazine par la 2-hydrazinopyridine.
*Spectre de masse :* ESI-MS : MH⁺ = 453

Les produits des exemples 80 à 96 sont obtenus selon le procédé décrit dans l'exemple 1 en remplaçant au stade a, les sulfonohydrazides par les hydrazides appropriés, et en utilisant les isothiocyanates et les hydrazines appropriées aux stades c et d, respectivement.

### EXEMPLE 80 : 4-[(1,5-Diphényl-1H-1,2,4-triazol-3-yl)amino]-N'-(3-nitrobenzoyl) benzohydrazide

*Spectre de masse :* ESI-MS : MH⁺ = 520

### EXEMPLE 81 : 4-[(1,5-Diphényl-1H-1,2,4-triazol-3-yl)amino]-N'-[4-(trifluorométhyl) benzoyl]benzohydrazide

*Spectre de masse :* ESI-MS : MH⁺ = 543

### EXEMPLE 82 : 4-[(1,5-Diphenyl-1H-1,2,4-triazol-3-yl)amino]-N'-(3,4,5-triméthoxybenzoyl)benzohydrazide

*Spectre de masse :* ESI-MS : MH⁺ = 565

### EXEMPLE 83: 4-[(1,5-Diphényl-1H-1,2,4-triazol-3-yl}amino]-N'-(3-méthoxybenzoyl) benzohydrazide

*Spectre de masse :* ESI-MS : MH⁺ = 505

### EXEMPLE 84: 4-[(1,5-Diphényl-1H-1,2,4-triazol-3-yl)amino]-N'-(4-méthoxybenzoyl) benzohydrazide

*Spectre de masse :* ESI-MS : MH⁺ = 505

### EXEMPLE 85 : 4-[(1,5-Diphényl-1H-1,2,4-triazol-3-yl)amino]-N'-(3-furoyl) benzohydrazide

*Spectre de masse :* ESI-MS : MH⁺ = 465

### EXEMPLE 86 : 4-[(1,5-Diphényl-1H-1,2,4-triazol-3-yl)amino]-N'-isonicotinoylbenzohydrazide

*Spectre de masse :* ESI-MS : MH⁺ = 476

### EXEMPLE 87 : 4-[(1,5-Diphényl-1H-1,2,4-triazol-3-yl)amino]-N'-(1-naphthoyl) benzohydrazide

*Spectre de masse :* ESI-MS : MH⁺ = 539

### EXEMPLE 88 : N'-(3,4-Diméthoxybenzoyl)-4-[(1,5-diphényl-1H-1,2,4-triazol-3-yl) amino]benzohydrazide

*Spectre de masse :* ESI-MS : MH⁺ = 535

### EXEMPLE 89: 4-[(1,5-Diphényl-1H-1,2,4-triazol-3-yl)amino]-N'-(2-naphthoyl) benzohydrazide

*Spectre de masse :* ESI-MS : MH⁺ = 525

### EXEMPLE 90 : N'-(3-chlorobenzoyl)-4-[(1,5-diphényl-1H-1,2,4-triazol-3-yl)amino] benzohydrazide

*Spectre de masse :* ESI-MS : MH⁺ = 509

### EXEMPLE 91 : N'-[3,5-bis(Trifluorométhyl)benzoyl]-4-[(1,5-diphényl-1H-1,2,4-triazol-3-yl)amino]benzohydrazide

*Spectre de masse :* ESI-MS : MH⁺ = 611

### EXEMPLE 92 : 4-[(1,5-Diphényl-1H-1,2,4-triazol-3-yl)amino]-N'-(2-thiénylcarbonyl) benzohydrazide

*Spectre de masse :* ESI-MS : MH⁺ = 481

### EXEMPLE 93 : N'-(4-Chlorobenzoyl)-4-[(1,5-diphényl-1H-1,2,4-triazol-3-yl)amino] benzohydrazide

*Spectre de masse :* ESI-MS : MH⁺ = 509

### EXEMPLE 94 : N'-(2-Chlorobenzoyl)-4-[(1,5-diphényl-1H-1,2,4-triazol-3-yl)amino] benzohydrazide

*Spectre de masse :* ESI-MS : MH⁺ = 509

### EXEMPLE 95 : 4-[(1,5-Diphényl-1H-1,2,4-triazol-3-yl)amino]-N'-(3-hydroxybenzoyl) benzohydrazide

*Spectre de masse :* ESI-MS : MH⁺ = 491

### EXEMPLE 96 : 4-[(1,5-Diphényl-1H-1,2,4-triazol-3-yl)amino]-N'-(3,4,5-trihydroxybenzoyl)benzohydrazide

*Spectre de masse :* ESI-MS : MH⁺ = 523

### EXEMPLE 97 : N'-{4-[(1-Benzyl-3-phényl-1H-1,2,4-triazol-5-yl)amino]benzoyl} benzènesulfonohydrazide

Le produit du titre est isolé au cours de la purification du composé décrit dans l'exemple 15.

### EXEMPLE 98 : N'-{4-[(1-Benzyl-3-phényl-1H-1,2,4-triazol-5-yl)amino]benzoyl}-4-méthoxybenzènesulfonohydrazide

Le produit du titre est isolé au cours de la purification du composé décrit dans l'exemple 25.

### ETUDE PHARMACOLOGIQUE

### EXEMPLE A : Mesure de l'effet sur la prise alimentaire et l'évolution pondérale chez la souris obèse

Les produits de l'invention ont été traités in vivo chez la souris obèse ob/ob, afin d'évaluer leur influence sur la prise alimentaire et l'évolution pondérale. Les animaux utilisés sont des souris C57B1/6J ob/ob femelles, âgées de 13 à 18 semaines. Ils sont répartis en lots de 4 animaux par cage équipées d'un sol grillagé et d'un accès libre à la nourriture.

Avant les expériences, les animaux sont conditionnés pendant une période variant de 2 à 3 semaines, jusqu'à une consommation alimentaire stabilisée. Le synoptique des expériences est le suivant :
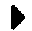 J - 14 à J - 7 : conditionnement
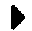 J - 7 à J -3 : mesure de la prise alimentaire basale
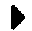 J0 à J + 3 : animaux traités 2 fois par jour, les lots contrôle recevant le véhicule
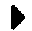 J0 à J + 4 : mesure quotidienne de la prise alimentaire et de poids corporel

Les produits testés sont mis en solution extemporanément dans l'eau, le chlorure de sodium à 0,9 %, le propylène glycol ou le diméthylsulfoxide en fonction de leur solubilité, et sont administrés par voie intrapéritonéale (IP), à un volume de 2,5 ml/kg.

Les paramètres mesurés sont la prise alimentaire et le poids corporel.

### Résultats :

Les résultats sont exprimés en :
- pourcentage de variation de la prise alimentaire sous traitement par rapport à la prise alimentaire basale ;
- pourcentage de variation du poids corporel entre le premier et le dernier jour de traitement.

A titre d'exemple, les résultats obtenus avec les composés des exemples 1 et 6 sont les suivants :

| Produit | Dose (mg/kg) | Prise alimentaire % variation (J1) | | Poids corporel % variation (J4/J0) |
|---|---|---|---|---|
| | | Contrôle | Traités | |
| Exemple 1 | 1 | - 34 | - 45 | - 6,4 |
| Exemple 6 | 5 | - 23 | - 34 | - 11,2 |

### EXEMPLE B : Mesure de l'affinité in vitro aux récepteurs du NPY

La capacité des composés de l'invention à se lier aux récepteurs du NPY a été mesurée sur différentes lignées cellulaires exprimant chacune un des sous-types réceptoriels étudiés. Des expériences de liaison compétitive ont été réalisées en utilisant comme radioligand le peptide [¹²⁵I]-PYY à des concentrations variant de 15 à 65 pM. La fraction non spécifique est mesurée en présence d'une concentration de 1 µM de NPY. Les cellules sont incubées pendant une période variant de 1 à 2 heures suivant les lignées, et la radioactivité est recueillie après filtration sur filtre GF/C traité par du PEI à 0,1 %, avant d'être mesurée.

### Résultats :

Les résultats sont exprimés en IC₅₀. Il apparaît que les composés de l'invention sont capables de déplacer de façon importante le ligand de référence : les IC₅₀ varient de quelques nanomoles à quelques centaines de nanomoles.

A titre d'exemple, les composés des exemples 1 et 6 possèdent des IC₅₀ de 80 nM et 7 nM respectivement pour le récepteur Y₅.

### EXEMPLE C : Etude de la toxicité aiguë

La toxicité aiguë a été appréciée après administration orale à des lots de 8 souris (26 ± 6 grammes) de doses croissantes de produit à étudier. Les animaux ont été observés à intervalles réguliers au cours de la première journée et quotidiennement pendant les deux semaines suivant le traitement.

Il apparaît que les composés de l'invention sont très peu toxiques.

### EXEMPLE D : Composition pharmaceutique

| Formule de préparation pour 1000 comprimés dosés à 10 mg | |
|---|---|
| Composé de l'exemple 1 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule (I) : 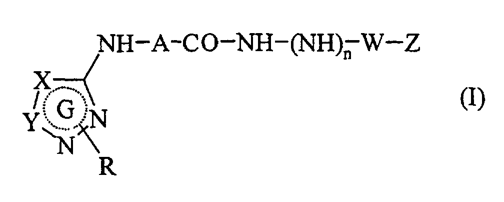 dans laquelle :
• n vaut 0 ou 1,
• W représente un groupement -CO- ou un groupement S(O)_{q} dans lequel q vaut 0, 1 ou 2,
• Le groupement 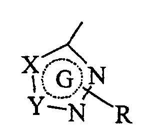 représente un groupement choisi parmi : 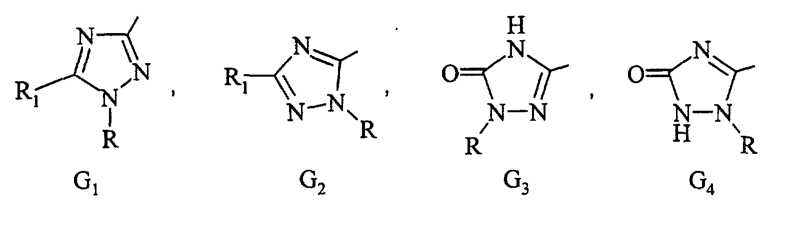
• Z représente un groupement alkyle, aryle éventuellement substitué, hétéroaryle éventuellement substitué, arylalkyle éventuellement substitué, arylalkényle éventuellement substitué, arylalkynyle éventuellement substitué, hétéroarylalkyle éventuellement substitué, hétéroarylalkényle éventuellement substitué ou hétéroarylalkynyle éventuellement substitué,
• A représente un groupement choisi parmi -A₂-, -A₁-A₂-, -A₂-A₁- ou -A₁-A₂-A₁- dans lesquels A₁ est un groupement alkylène et A₂ représente un groupement phénylène éventuellement substitué, naphtylène éventuellement substitué, cycloalkylène, ou hétéroarylène éventuellement substitué,
• R représente un atome d'hydrogène, un groupement alkyle, aryle éventuellement substitué, hétéroaryle éventuellement substitué, arylalkyle éventuellement substitué, arylalkényle éventuellement substitué, arylalkynyle éventuellement substitué, hétéroarylalkyle éventuellement substitué, hétéroarylalkényle éventuellement substitué, ou hétéroarylalkynyle éventuellement substitué,
• R₁ représente un groupement alkyle, aryle éventuellement substitué, hétéroaryle éventuellement substitué, arylalkyle éventuellement substitué, arylalkényle éventuellement substitué, arylalkynyle éventuellement substitué, hétéroarylalkyle éventuellement substitué, hétéroarylalkényle éventuellement substitué, ou hétéroarylalkynyle éventuellement substitué,
leurs énantiomères, diastéréoisomères, ainsi que leurs sels d'addition à un acide ou à une base pharmaceutiquement acceptable,
étant entendu que,
- le terme alkyle désigne un groupement linéaire ou ramifié de 1 à 6 atomes de carbone,
- le terme alkylène désigne un radical bivalent, linéaire ou ramifié, contenant de 1 à 6 atomes de carbone,
- le terme alkényle désigne un groupement linéaire ou ramifié contenant de 2 à 6 atomes de carbone et de 1 à 3 double liaison,
- le terme alkynyle désigne un groupement linéaire ou ramifié contenant de 2 à 6 atomes de carbone et de 1 à 3 triple liaison,
- le terme aryle désigne un groupement phényle, napthyle, biphényle, dihydronapthyle, ou tétrahydronapthyle,
- le terme hétéroaryle désigne un groupement mono ou bicyclique insaturé ou partiellement insaturé, de 5 à 11 chaînons, contenant de 1 à 4 hétéroatomes choisis parmi azote, oxygène et soufre.
- les termes phénylène et naphtylène désignent respectivement des radicaux phényle et naphtyle bivalents,
- le terme cycloalkylène désigne un groupement bivalent cyclique saturé contenant de 3 à 8 atomes de carbone,
- le terme hétéroarylène désigne un radical bivalent hétéroaryle tel que défini précédemment,
l'expression "éventuellement substitué" affectée aux termes aryle, arylalkyle, hétéroaryle, ou hétéroarylalkyle, signifie que ces groupements sont substitués sur leur partie cyclique par 1 à 5 substituants, identiques ou différents, choisis parmi alkyle (C₁-C₆) linéaire ou ramifié, alkoxy (C₁-C₆) linéaire ou ramifié, halogène, hydroxy, perhalogénoalkyle (C₁-C₆) linéaire ou ramifié, nitro, amino (éventuellement substitué par un ou deux groupements alkyle (C₁-C₆) linéaire ou ramifié), acyle (C₁-C₆) linéaire ou ramifié, aminocarbonyle (éventuellement substitué sur l'atome d'azote par un ou deux groupements alkyle (C₁-C₆) linéaire ou ramifié), acylamino (C₁-C₆) linéaire ou ramifié, alkoxycarbonyle (C₁-C₆) linéaire ou ramifié, formyle, carboxy, sulfo, nitrile, aminoalkyle (C₁-C₆) linéaire ou ramifié (éventuellement substitué sur l'atome d'azote par un ou deux groupements alkyle (C₁-C₆) linéaire ou ramifié), thioalkyle (C₁-C₆) linéaire ou ramifié (éventuellement substitué sur l'atome de soufre par un groupement alkyle (C₁-C₆) linéaire ou ramifié), ou hydroxyalkyle (éventuellement substitué sur l'atome d'oxygène par un groupement alkyle (C₁-C₆) linéaire ou ramifié),
l'expression "éventuellement substitué" affectée aux termes phénylène, naphtylène ou hétéroarylène signifie que ces groupements sont substitués par un à trois groupements identiques ou différents, choisis parmi alkyle (C₁-C₆) linéaire ou ramifié, alkoxy (C₁-C₆) linéaire ou ramifié, halogène, hydroxy, perhalogénoalkyle (C₁-C₆) linéaire ou ramifié, nitro, amino (éventuellement substitué par un ou deux groupements alkyle (C₁-C₆) linéaire ou ramifié), acyle (C₁-C₆) linéaire ou ramifié, formyle, carboxy, alkoxycarbonyle (C₁-C₆) linéaire ou ramifié, aminocarbonyle (éventuellement substitué sur l'atome d'azote par un ou deux groupements alkyle (C₁-C₆) linéaire ou ramifié), acylamino (C₁-C₆) linéaire ou ramifié, ou nitrile.

2. Composés de formule (I) selon la revendication 1 pour lesquels n vaut 1, leurs énantiomères, diastéréoisomères ainsi que leurs sels d'addition à un acide ou à une base pharmaceutiquement acceptable.

3. Composés de formule (I) selon la revendication 1 pour lesquels n vaut 0, leurs énantiomères, diastéréoisomères ainsi que leurs sels d'addition à un acide ou à une base pharmaceutiquement acceptable.

4. Composés de formule (I) selon la revendication 1 pour lesquels W représente un groupement SO₂, leurs énantiomères, diastéréoisomères ainsi que leurs sels d'addition à un acide ou à une base pharmaceutiquement acceptable.

5. Composés de formule (I) selon la revendication 1 pour lesquels le groupement 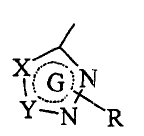 représente un groupement choisi parmi : 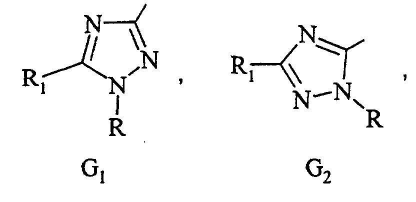 leurs énantiomères, diastéréoisomères ainsi que leurs sels d'addition à un acide ou à une base pharmaceutiquement acceptable.

6. Composés de formule (I) selon la revendication 1 pour lesquels le groupement 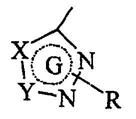 représente un groupement choisi parmi : 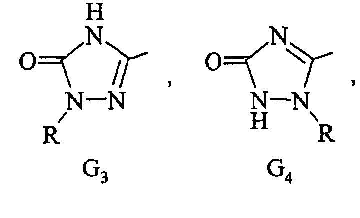 leurs énantiomères, diastéréoisomères ainsi que leurs sels d'addition à un acide ou à une base pharmaceutiquement acceptable.

7. Composés de formule (I) selon la revendication 1 pour lesquels A représente un groupement A₂, leurs énantiomères, diastéréoisomères ainsi que leurs sels d'addition à un acide ou à une base pharmaceutiquement acceptable.

8. Composés de formule (I) selon la revendication 1 pour lesquels A représente un groupement -A₁-A₂-, ou -A₂-A₁-, leurs énantiomères, diastéréoisomères ainsi que leurs sels d'addition à un acide ou à une base pharmaceutiquement acceptable.

9. Composés de formule (I) selon la revendication 1 pour lesquels R₁ représente un groupement aryle éventuellement substitué, leurs énantiomères, diastéréoisomères ainsi que leurs sels d'addition à un acide ou à une base pharmaceutiquement acceptable.

10. Composés de formule (I) selon la revendication 1 pour lesquels R est choisi parmi un atome d'hydrogène, un groupement aryle éventuellement substitué, ou un groupement hétéroaryle éventuellement substitué, leurs énantiomères, diastéréoisomères ainsi que leurs sels d'addition à un acide ou à une base pharmaceutiquement acceptable.

11. Composés de formule (I) selon la revendication 1 pour lesquels Z représente un groupement choisi parmi alkyle, aryle éventuellement substitué, et hétéroaryle éventuellement substitué, leurs énantiomères, diastéréoisomères ainsi que leurs sels d'addition à un acide ou à une base pharmaceutiquement acceptable.

12. Composés de formule (I) selon la revendication 1 pour lesquels n vaut 1, W représente un groupement SO₂, A représente un groupement choisi parmi phénylène, pyridinylène, et pyrazinylène, R₁ représente un groupement aryle éventuellement substitué, R est choisi parmi un atome d'hydrogène, un groupement aryle éventuellement substitué et hétéroaryle éventuellement substitué, et Z représente un groupement alkyle, aryle éventuellement substitué ou hétéroaryle éventuellement substitué, leurs énantiomères, diastéréoisomères ainsi que leurs sels d'addition à un acide ou à une base pharmaceutiquement acceptable.

13. Composé de formule (I) selon la revendication 1 qui est le N'-[4-({5-phényl-1-[3-(trifluorométhyl)phényl]-*1H*-1,2,4-triazol-3-yl}amino)benzoyl]benzènesulfono hydrazide.

14. Composé de formule (I) selon la revendication 1 qui est le N'-{4-[(5-phényl-1*H*-1,2,4-triazol-3-yl)amino]benzoyl}benzènesulfonohydrazide.

15. Composé de formule (I) selon la revendication 1 qui est le N'-(4-{[5-phényl-1-(2-pyridyl)-1*H*-1,2,4-triazol-3-yl)amino}benzoyl)benzènesulfonohydrazide.

16. Composé de formule (I) selon la revendication 1 qui est le N'-[4-({5-oxo-1-[3-(trifluorométhyl)phényl]-4,5-dihydro-*1H*-1,2,4-triazol-3-yl}amino)benzoyl}benzène sulfonohydrazide.

17. Composé de formule (I) selon la revendication 1 qui est le N'-(4-{[5-oxo-1-(2-pyridyl)-4,5-dihydro-*1H*-1,2,4-triazol-3-yl]amino}benzoyl)benzènesulfonohydrazide.

18. Composé de formule (I) selon la revendication 1 qui est le N'-[(6-{[5-oxo-1-(2-pyridinyl)-4,5-dihydro-*1H*-1,2,4-triazol-3-yl]amino}-3-pyridinyl)carbonyl]benzène sulfonohydrazide.

19. Procédé de préparation des composés de formule (I) selon la revendication 1 **caractérisé en ce que** l'on utilise comme produit de départ un composé de formule (II) : 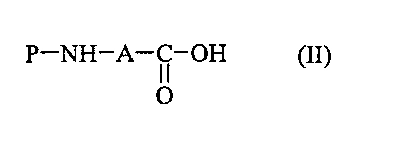 dans laquelle A a la même définition que dans la formule (I) et P représente un groupement protecteur de la fonction amine,
qui réagit en présence d'un agent de couplage avec un composé de formule (III) :
NH₂-(NH)ₙ-W-Z (III)
dans laquelle n, W et Z sont tels que définis dans la formule (I),
pour conduire, après déprotection de la fonction amine par clivage du groupement P, à un composé de formule (IV) : 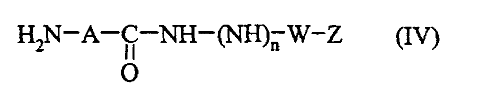 dans laquelle n, A, W et Z sont tels que définis précédemment,
composé (IV) qui est ensuite condensé en milieu basique sur un isothiocyanate de formule (V) : 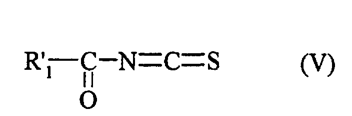 dans laquelle R'₁ a la même signification que R₁ dans la formule (I) ou bien représente un groupement alkoxy (C₁-C₆) linéaire ou ramifié,
pour conduire à un composé de formule (VI) : 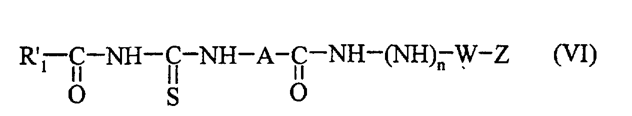 dans laquelle n, R'₁, A, W et Z sont tels que définis précédemment,
composé de formule (VI) qui est :
- soit, lorsque R'₁ représente un groupement alkoxy (C₁-C₆) linéaire ou ramifié, condensé en présence d'un agent de couplage sur une hydrazine de formule R-NH-NH₂, dans laquelle R est tel que défini dans la formule (I), pour conduire à un composé de formule (VII/a) : 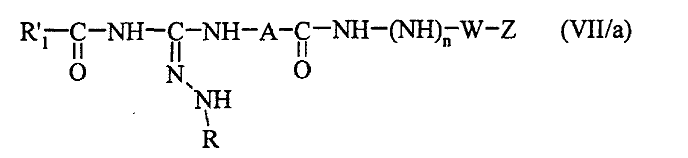
dans laquelle R, A, n, W et Z sont tels que définis précédemment, et R'₁ représente un groupement alkoxy (C₁-C₆) linéaire ou ramifié,
composé (VII/a) qui se cyclise, spontanément ou après traitement en milieu acide, suivant la nature du groupement R, pour conduire au mélange des deux composés de formule (I/a) et (I/b) : 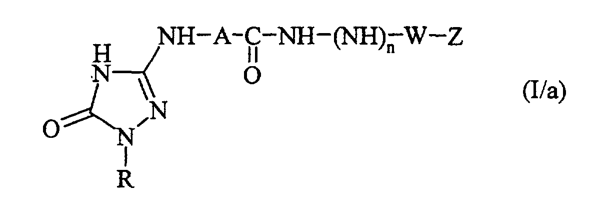 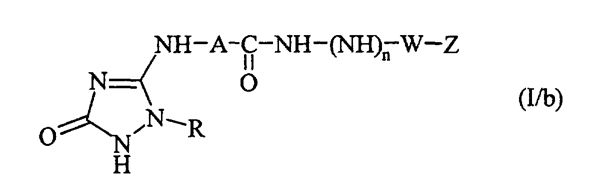 cas particuliers des composés de formule (I) pour lesquels R, A, n, W et Z sont tels
que définis précédemment,
composés (I/a) et (I/b) qui peuvent être séparés selon des techniques classiques de séparation,
- soit, lorsque R'₁ représente un groupement R₁ tel que défini dans la formule (I), condensé en présence d'un agent de couplage sur une hydrazine de formule R-NH-NH₂, dans laquelle R est tel que défini dans la formule (I), pour conduire à un composé de formule (VII/b) : 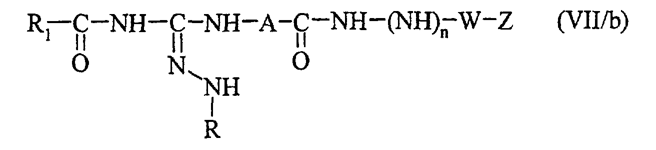
dans laquelle R₁, R, A, n, W et Z sont tels que définis précédemment,
composé (VII/b) qui subit une réaction de cyclisation suivie d'une deshydration, de façon spontanée ou après traitement en milieu acide, suivant la nature du groupement R, pour conduire au mélange des deux composés de formule (I/c) et (I/d) : 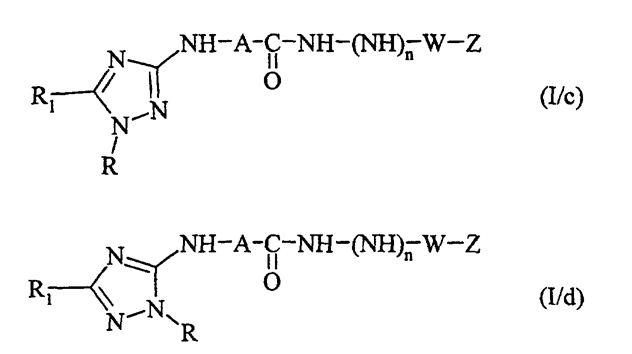 cas particuliers des composés de formule (I) pour lesquels R₁, R, A, n, W et Z sont tels que définis précédemment,
composés (I/c) et (I/d) qui peuvent être séparés selon des techniques classiques de séparation,
les composés (I/a), (I/b), (I/c) et (I/d) formant la totalité des composés de formule (I),
- dont on sépare, le cas échéant, les énantiomères et/ou diastéréoisomères selon une technique classique de séparation,
- que l'on transforme, si on le souhaite, en leurs sels d'addition à un acide ou à une base pharmaceutiquement acceptable.

20. Compositions pharmaceutiques contenant comme principe actif au moins un composé selon l'une quelconque des revendications 1 à 18, seul ou en combinaison avec un ou plusieurs excipients ou véhicules inertes, non toxiques, pharmaceutiquement acceptables.

21. Compositions pharmaceutiques selon la revendication 20 contenant au moins un principe actif selon l'une quelconque des revendications 1 à 18 utiles, comme ligand des récepteurs du Neuropeptide Y, dans le traitement des pathologies liées à des troubles du comportement alimentaire ou de la balance énergétique, telles que le diabète, l'obésité, la boulimie, l'anorexie nerveuse, ou bien dans le traitement de l'hypertension artérielle, de l'anxiété, des dépressions de l'épilepsie, des dysfonctionnements sexuels et des troubles du sommeil.

## Patentansprüche

1. Verbindungen der Formel (I): 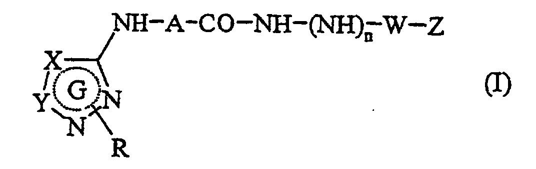 in der:
• n 0 oder 1 bedeutet,
• W eine Gruppe -CO- oder eine Gruppe S(O)_{q} bedeutet, worin q 0, 1 oder 2 darstellt,
• die Gruppe 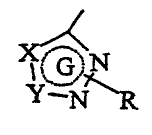 eine Gruppe ausgewählt aus: 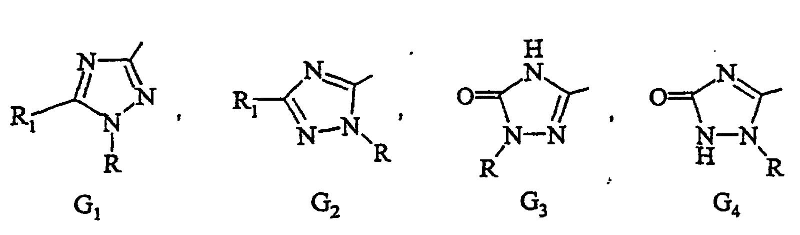 darstellt,
• Z eine Alkylgruppe, gegebenenfalls substituierte Arylgruppe, gegebenenfalls substituierte Heteroarylgruppe, gegebenenfalls substituierte Arylalkylgruppe, gegebenenfalls substituierte Arylalkenylgruppe, gegebenenfalls substituierte Arylalkinylgruppe, gegebenenfalls substituierte Heteroarylalkylgruppe, gegebenenfalls substituierte Heteroarylalkenylgruppe oder gegebenenfalls substituierte Heteroarylalkinylgruppe bedeutet,
• A eine Gruppe ausgewählt aus -A₂-, -A₁-A₂-, -A₂-A₁- oder -A₁-A₂-A₁- darstellt, worin A₁ eine Alkylengruppe und A₂ eine gegebenenfalls substituierte Phenylengruppe, gegebenenfalls substituierte Naphthylengruppe, Cycloalkylengruppe oder gegebenenfalls substituierte Heteroarylengruppe darstellen,
• R ein Wasserstoffatom, eine Alkylgruppe, gegebenenfalls substituierte Arylgruppe, gegebenenfalls substituierte Heteroarylgruppe, gegebenenfalls substituierte Arylalkylgruppe, gegebenenfalls substituierte Arylalkenylgruppe, gegebenenfalls substituierte Arylalkinylgruppe, gegebenenfalls substituierte Heteroarylalkylgruppe, gegebenenfalls substituierte Heteroarylalkenylgruppe oder gegebenenfalls substituierte Heteroarylalkinylgruppe bedeutet,
• R₁ eine Alkylgruppe, gegebenenfalls substituierte Arylgruppe, gegebenenfalls substituierte Heteroarylgruppe, gegebenenfalls substituierte Arylalkylgruppe, gegebenenfalls substituierte Arylalkenylgruppe, gegebenenfalls substituierte Arylalkinylgruppe, gegebenenfalls substituierte Heteroarylalkylgruppe, gegebenenfalls substituierte Heteroarylalkenylgruppe oder gegebenenfalls substituierte Heteroarylalkinylgruppe bedeutet,
deren Enantiomere, Diastereoisomere sowie deren Additionssalze mit einer pharmazeutisch annehmbaren Säure oder Base,
wobei es sich versteht, daß
- der Begriff Alkyl für eine geradkettige oder verzweigte Gruppe mit 1 bis 6 Kohlenstoffatomen steht,
- der Begriff Alkylen für eine zweiwertige, geradkettige oder verzweigte Gruppe steht, die 1 bis 6 Kohlenstoffatome enthält,
- der Begriff Alkenyl für eine geradkettige oder verzweigte Gruppe steht, die 2 bis 6 Kohlenstoffatome und 1 bis 3 Doppelbindungen aufweist,
- der Begriff Alkinyl für eine geradkettige oder verzweigte Gruppe steht, die 2 bis 6 Kohlenstoffatome und 1 bis 3 Dreifachbindungen aufweist.
- der Begriff Aryl für eine Phenyl-, Naphthyl-, Biphenyl-, Dihydronaphthyl- oder Tetrahydronaphthylgruppe steht,
- der Begriff Heteroaryl für eine mono- oder bicyclische, ungesättigte oder teilweise ungesättigte Gruppe mit 5 bis 11 Kettengliedern steht, die 1 bis 4 Heteroatome ausgewählt aus Stickstoff, Sauerstoff und Schwefel enthält,
- die Begriffe Phenylen und Naphthylen für zweiwertige Phenyl- bzw. Naphthylreste stehen,
- der Begriff Cycloalkylen für eine zweiwertige gesättigte cyclische Gruppe steht, die 3 bis 8 Kohlenstoffatome aufweist.
- der Begriff Heteroarylen für einen zweiwertigen Heteroarylrest steht, wie er oben definiert worden ist,
der Begriff "gegebenenfalls substituiert" in bezug auf die Begriffe Aryl, Arylalkyl, Heteroaryl oder Heteroarylalkyl bedeutet, daß diese Gruppen an ihrem cyclischen Teil durch 1 bis 5 gleichartige oder verschiedene Substituenten substituiert sind, ausgewählt aus geradkettigem oder verzweigtem (C₁-C₆)-Alkyl, geradkettigem oder verzweigtem (C₁-C₆)-Alkoxy, Halogen, Hydroxy, geradkettigem oder verzweigtem (C₁-C₆)-Perhalogenalkyl, Nitro, Amino (gegebenenfalls durch eine oder zwei geradkettige oder verzweigte (C₁-C₆)-Alkylgruppen substituiert), geradkettigem oder verzweigtem (C₁-C₆)-Acyl, Aminocarbonyl (gegebenenfalls am Stickstoffatom durch eine oder zwei geradkettige oder verzweigte (C₁-C₆)-Alkylgruppen substituiert), geradkettigem oder verzweigtem (C₁-C₆)-Acylamino, geradkettigem oder verzweigtem (C₁-C₆)-Alkoxycarbonyl, Formyl, Carboxy, Sulfo, Nitril, geradkettigem oder verzweigtem (C₁-C₆)-Aminoalkyl (gegebenenfalls am Stickstoffatom durch eine oder zwei geradkettige oder verzweigte (C₁-C₆)-Alkylgruppen substituiert), geradkettigem oder verzweigtem (C₁-C₆)-Thioalkyl (gegebenenfalls am Schwefelatom durch eine geradkettige oder verzweigte (C₁-C₆)-Alkylgruppe substituiert) oder Hydroxyalkyl (gegebenenfalls am Sauerstoffatom durch eine geradkettige oder verzweigte (C₁-C₆)-Alkylgruppe substituiert),
der Begriff "gegebenenfalls substituiert", bezogen auf die Begriffe Phenylen, Naphthylen oder Heteroarylen bedeutet, daß diese Gruppen durch eine bis drei gleichartige oder verschiedene Gruppen substituiert sind ausgewählt aus geradkettigem oder verzweigtem (C₁-C₆)-Alkyl, geradkettigem oder verzweigtem (C₁-C₆)-Alkoxy, Halogen, Hydroxy, geradkettigem oder verzweigtem (C₁-C₆)-Perhalogenalkyl, Nitro, Amino (gegebenenfalls durch eine oder zwei geradkettige oder verzweigte (C₁-C₆)-Alkylgruppen substituiert), geradkettigem oder verzweigtem (C₁-C₆)-Acyl, Formyl, Carboxy, geradkettigem oder verzweigtem (C₁-C₆)-Alkoxycarbonyl, Aminocarbonyl (gegebenenfalls am Stickstoffatom durch eine oder zwei geradkettige oder verzweigte (C₁-C₆)-Alkylgruppen substituiert), geradkettigem oder verzweigtem (C₁-C₆)-Acylamino und Nitril.

2. Verbindungen der Formel (I) nach Anspruch 1, worin n 1 bedeutet, deren Enantiomere, Diastereoisomere sowie deren Additionssalze mit einer pharmazeutisch annehmbaren Säure oder Base.

3. Verbindungen der Formel (I) nach Anspruch 1, worin n 0 bedeutet, deren Enantiomere, Diastereoisomere sowie deren Additionssalze mit einer pharmazeutisch annehmbaren Säue oder Base.

4. Verbindungen der Formel (I) nach Anspruch 1, worin W eine Gruppe SO₂ bedeutet, deren Enantiomere, Diastereoisomere sowie deren Additionssalze mit einer pharmazeutisch annehmbaren Säure oder Base.

5. Verbindungen der Formel (I) nach Anspruch 1, worin die Gruppe 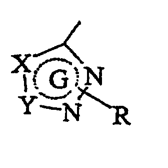 eine Gruppe bedeutet ausgewählt aus: 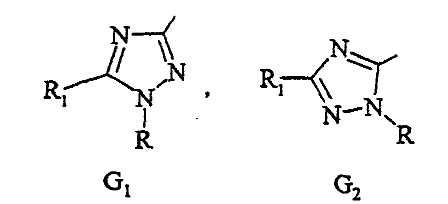 deren Enantiomere, Diastereoisomere sowie deren Additionssalze mit einer pharmazeutisch annehmbaren Säure oder Base.

6. Verbindungen der Formel (I) nach Anspruch 1, worin die Gruppe 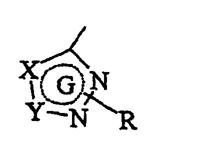 eine Gruppe ausgewählt aus: 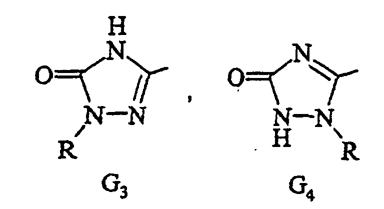 bedeutet, deren Enantiomere, Diastereoisomere sowie deren Additionssalze mit einer pharmazeutisch annehmbaren Säure oder Base.

7. Verbindungen der Formel (I) nach Anspruch 1, worin A eine Gruppe A₂ darstellt, deren Enantiomere, Diastereoisomere sowie deren Additionssalze mit einer pharmazeutisch annehmbaren Säure oder Base.

8. Verbindungen der Formel (I) nach Anspruch 1, worin A eine Gruppe -A₁-A₂- oder -A₂-A₁- bedeutet, deren Enantiomere, Diastereoisomere sowie deren Additionssalze mit einer pharmazeutisch annehmbaren Säure oder Base.

9. Verbindungen der Formel (I) nach Anspruch 1, worin R₁ eine gegebenenfalls substituierte Arylgruppe bedeutet, deren Enantiomere, Diastereoisomere sowie deren Additionssalze mit einer pharmazeutisch annehmbaren Säure oder Base.

10. Verbindungen der Formel (I) nach Anspruch 1, worin R aus Wasserstoffatomen, gegebenenfalls substituierten Arylgruppen oder gegebenenfalls substituierten Heteroarylgruppen ausgewählt ist, deren Enantiomere, Diastereoisomere sowie deren Additionssalze mit einer pharmazeutisch annehmbaren Säure oder Base.

11. Verbindungen der Formel (I) nach Anspruch 1, worin Z eine Gruppe ausgewählt aus Alkyl, gegebenenfalls substituiertem Aryl und gegebenenfalls substituiertem Heteroaryl bedeutet, deren Enantiomere, Diastereoisomere sowie deren Additionssalze mit einer pharmazeutisch annehmbaren Säure oder Base.

12. Verbindungen der Formel (I) nach Anspruch 1, worin n 1, W eine Gruppe SO₂, A eine Gruppe ausgewählt aus Phenylen, Pyridinylen und Pyrazinylen, R₁ eine gegebenenfalls substituierte Arylgruppe, R eine Gruppe ausgewählt aus Wasserstoff, gegebenenfalls substituierten Arylgruppen und gegebenenfalls substituierten Heteroarylgruppen und Z eine Alkylgruppe, gegebenenfalls substituierte Arylgruppe oder gegebenenfalls substituierte Heteroarylgruppe bedeuten, deren Enantiomere, Diastereoisomere sowie deren Additionssalze mit einer pharmazeutisch annehmbaren Säure oder Base.

13. Verbindung der Formel (I) nach Anspruch 1, nämlich N'-[4-({5-Phenyl-1-[3-(trifluormethyl)-phenyl]-1*H*-1,2,4-triazol-3-yl}-amino)-benzoyl]-benzolsulfonohydrazid.

14. Verbindung der Formel (I) nach Anspruch 1, nämlich N'-{4-[(5-Phenyl-1*H*-1,2,4-triazol-3-yl]-amino]-benzoyl)-benzolsulfonohydrazid.

15. Verbindung der Formel (I) nach Anspruch 1, nämlich N'-(4-{[5-Phenyl-1-(2-pyridyl)-1*H*-1,2,4-triazol-3-yl]-amino}-benzoyl)-benzolsulfonohydrazid.

16. Verbindung der Formel (I) nach Anspruch 1, nämlich N'-[4-({5-Oxo-1-[3-(trifluormethyl)-phenyl]-4,5-dihydro-1*H*-1,2,4-triazol-3-yl}-amino)-benzoyl]-benzolsulfonohydrazid.

17. Verbindung der Formel (I) nach Anspruch 1, nämlich N'-(4-{[5-Oxo-1-(2-pyridyl)-4,5-dihydro-1*H*-1,2,4-triazol-3-yl]-amino}-benzoyl)-benzolsulfonohydrazid.

18. Verbindung der Formel (I) nach Anspruch 1, nämlich N'-[(6-{[5-Oxo-1-(2-pyridinyl)-4,5-dihydro-1*H*-1,2,4-triazol-3-yl]-amino}-3-pyridinyl]-carbonyl]-benzolsulfonohydrazid.

19. Verfahren zur Herstellung der Verbindungen der Formel (I) nach Anspruch 1, **dadurch gekennzeichnet, daß** man als Ausgangsprodukt eine Verbindung der Formel (II) verwendet: 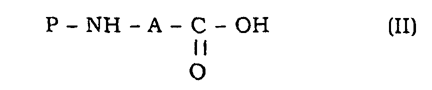 in der A die bezüglich der Formel (I) angegebenen Bedeutungen besitzt und P eine Schutzgruppe für die Aminfunktion darstellt, welche in Gegenwart eines Kupplungsmittels mit einer Verbindung der Formel (III):
NH₂ - (NH)ₙ- W - Z (III)
in der n, W und Z die bezüglich der Formel (I) angegebenen Bedeutungen besitzen,
reagiert, so daß man nach der Abspaltung der Schutzgruppe der Aminfunktion durch Abspalten der Gruppe P eine Verbindung der Formel (IV) erhält: 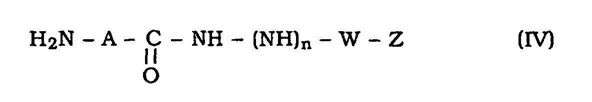 in der n, A, W und Z die oben angegebenen Bedeutungen besitzen,
welche Verbindung (IV) anschließend in basischem Medium mit einem Isothiocyanat der Formel (V) kondensiert wird: 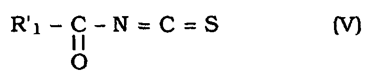 in der R'₁ die gleichen Bedeutungen besitzt wie R₁ der Formel (I) oder eine geradkettige oder verzweigte (C₁-C₆)-Alkoxygruppe darstellt,
zur Bildung einer Verbindung der Formel (VI): 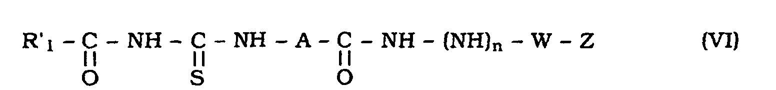 in der n, R'₁, A, W und Z die oben angegebenen Bedeutungen besitzen, welche Verbindung der Formel (VI):
- entweder, wenn R'₁ eine geradkettige oder verzweigte (C₁-C₆)-Alkoxygruppe darstellt, in Gegenwart eines Kupplungsmittels mit einem Hydrazin der Formel R-NH-NH₂, in der R die bezüglich der Formel (I) angegebenen Bedeutungen besitzt, kondensiert wird zur Bildung einer Verbindung der Formel (VII/a): 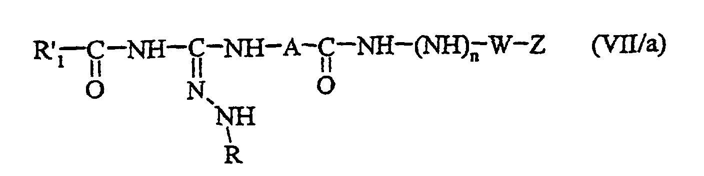
in der R, A, n, W und Z die oben angegebenen Bedeutungen besitzen und R'₁ eine geradkettige oder verzweigte (C₁-C₆)-Alkoxygruppe bedeutet,
welche Verbindung (VII/a) sich spontan oder nach der Behandlung in saurem Medium in Abhängigkeit von der Art der Gruppe R cyclisiert zur Bildung einer Mischung der beiden Verbindungen der Formeln (I/a) und (I/b): 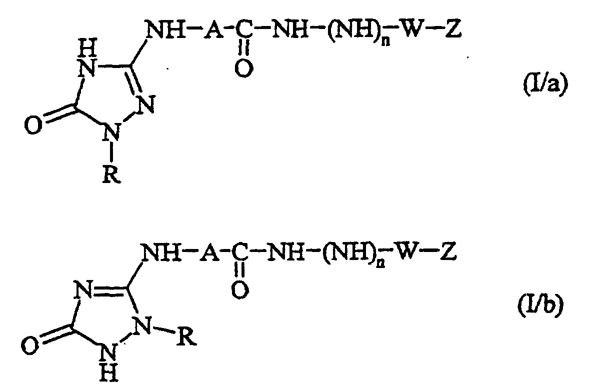 Sonderfällen der Verbindungen der Formel (I), worin R, A, n, W und Z die oben
angegebenen Bedeutungen besitzen,
welche Verbindungen (I/a) und (I/b) mit Hilfe klassischer Trennmethoden getrennt werden können,
- oder, wenn R'₁ eine Gruppe R₁ bedeutet, wie sie für die Formel (I) definiert worden ist, in Gegenwart eines Kupplungsmittels mit einem Hydrazin der Formel R-NH-NH₂, worin R die bezüglich der Formel (I) angegebenen Bedeutungen besitzt, kondensiert wird zur Bildung einer Verbindung der Formel (VII/b): 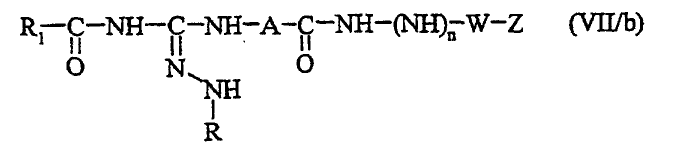
in der R₁, R, A, n, W und Z die oben angegebenen Bedeutungen besitzen, welche Verbindung (VII/b) einer Cyclisierungsreaktion gefolgt von einer Dehydratisierung in spontaner Weise oder nach der Behandlung in saurem Medium, in Abhängigkeit von der Art der Gruppe R unterliegt, zur Bildung einer Mischung der beiden Verbindungen der Formeln (I/c) und (I/d): 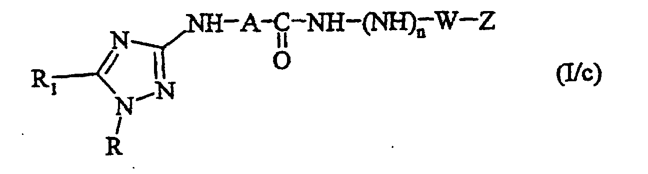 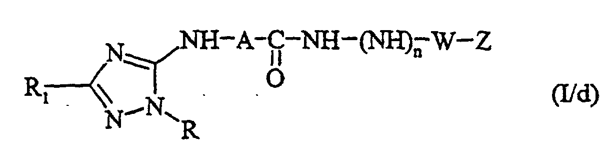 Sonderfällen der Verbindungen der Formel (I), worin R₁, R, A, n, W und Z die
oben angegebenen Bedeutungen besitzen,
welche Verbindungen (I/c) und (I/d) mit Hilfe klassischer Trennungsmethoden getrennt werden können,
welche Verbndungen (I/a), (I/b), (I/c) und (I/d) die Gesamtheit der Verbindungen der Formel (I) bilden,
- welche man gegebenenfalls mit Hilfe einer klassischen Trennmethode in ihre Enantiomeren und/oder Diastereoisomeren trennt,
- oder man gewünschtenfalls in ihre Additionssalze mit einer pharmazeutisch annehmbaren Säure oder Base überführt.

20. Pharmazeutische Zubereitungen enthaltend als Wirkstoff mindestens eine Verbindung nach einem der Ansprüche 1 bis 18 allein oder in Kombination mit einem oder mehreren inerten, nichttoxischen, pharmazeutisch annehmbaren Trägermaterialien oder Bindemitteln.

21. Pharmazeutische Zubereitungen nach Anspruch 20 enthaltend mindestens einen Wirkstoff nach einem der Ansprüche 1 bis 18, die nützlich sind als Liganden für die Rezeptoren des Neuropeptids Y, bei der Behandlung von pathologischen Zuständen, die mit Störungen des Ernährungsverhaltens oder der Energiebilanz verknüpft sind, wie des Diabetes, der Fettsucht, der Bulimie, der nervösen Anorexie oder bei der Behandlung der arteriellen Hypertension, der Angst, von Depressionen der Epilepsie, sexuellen Dysfunktionen und Schlafstörungen.

## Claims

1. Compounds of formula (I): 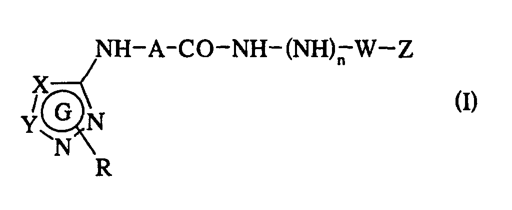 wherein :
• n is 0 or 1,
• W represents a -CO- group or an S(O)_{q} group wherein q is 0, 1 or 2,
• the grouping 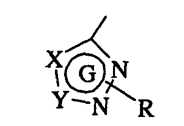 represents a group selected from: 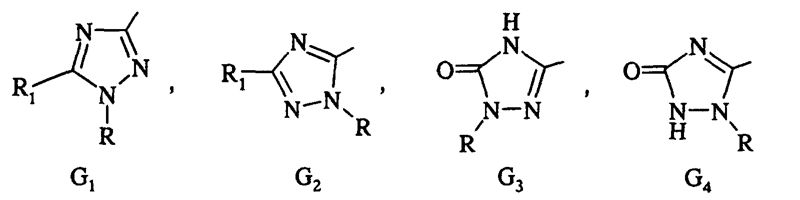
• Z represents an alkyl, optionally substituted aryl, optionally substituted heteroaryl, optionally substituted arylalkyl, optionally substituted arylalkenyl, optionally substituted arylalkynyl, optionally substituted heteroarylalkyl, optionally substituted heteroarylalkenyl or optionally substituted heteroarylalkynyl group,
• A represents a grouping selected from -A₂-, -A₁-A₂-, -A₂-A₁- and -A₁-A₂-A₁- wherein A₁ is an alkylene group and A₂ represents an optionally substituted phenylene, optionally substituted naphthylene, cycloalkylene or optionally substituted heteroarylene group,
• R represents a hydrogen atom, or an alkyl, optionally substituted aryl, optionally substituted heteroaryl, optionally substituted arylalkyl, optionally substituted arylalkenyl, optionally substituted arylalkynyl, optionally substituted heteroarylalkyl, optionally substituted heteroarylalkenyl or optionally substituted heteroarylalkynyl group,
• R₁ represents an alkyl, optionally substituted aryl, optionally substituted heteroaryl, optionally substituted arylalkyl, optionally substituted arylalkenyl, optionally substituted arylalkynyl, optionally substituted heteroarylalkyl, optionally substituted heteroarylalkenyl or optionally substituted heteroarylalkynyl group,
their enantiomers, diastereoisomers, and addition salts thereof with a pharmaceutically acceptable acid or base,
it being understood that
- the term "alkyl" denotes a linear or branched group having from 1 to 6 carbon atoms,
- the term "alkylene" denotes a linear or branched bivalent radical containing from 1 to 6 carbon atoms,
- the term "alkenyl" denotes a linear or branched group containing from 2 to 6 carbon atoms and from 1 to 3 double bonds,
- the term "alkynyl" denotes a linear or branched group containing from 2 to 6 carbon atoms and from 1 to 3 triple bonds,
- the term "aryl" denotes a phenyl, naphthyl, biphenyl, dihydronaphthyl or tetrahydronaphthyl group,
- the term "heteroaryl" denotes an unsaturated or partially unsaturated mono- or bi-cyclic group having from 5 to 11 ring members, containing from 1 to 4 hetero atoms selected from nitrogen, oxygen and sulphur,
- the terms "phenylene" and "naphthylene" denote bivalent phenyl and naphthyl radicals, respectively,
- the term "cycloalkylene" denotes a bivalent saturated cyclic group containing from 3 to 8 carbon atoms,
- the term "heteroarylene" denotes a bivalent heteroaryl radical as defined hereinbefore,
the expression "optionally substituted" applied to the terms "aryl", "arylalkyl", "heteroaryl" or "heteroarylalkyl" means that those groups are substituted on their cyclic moiety by from 1 to 5 identical or different substituents selected from linear or branched (C₁-C₆)alkyl, linear or branched (C₁-C₆)alkoxy, halogen, hydroxy, linear or branched perhalo-(C₁-C₆)alkyl, nitro, amino (optionally substituted by one or two linear or branched (C₁-C₆)alkyl groups), linear or branched (C₁-C₆)acyl, aminocarbonyl (optionally substituted on the nitrogen atom by one or two linear or branched (C₁-C₆)alkyl groups), linear or branched (C₁-C₆)acylamino, linear or branched (C₁-C₆)alkoxycarbonyl, formyl, carboxy, sulpho, nitrile, amino-(C₁-C₆)alkyl in which the alkyl moiety may be linear or branched (optionally substituted on the nitrogen atom by one or two linear or branched (C₁-C₆)alkyl groups), mercapto-(C₁-C₆)alkyl in which the alkyl moiety may be linear or branched (optionally substituted on the sulphur atom by a linear or branched (C₁-C₆)alkyl group), and hydroxyalkyl (optionally substituted on the oxygen atom by a linear or branched (C₁-C₆)alkyl group),
the expression "optionally substituted" applied to the terms "phenylene", "naphthylene" or "heteroarylene" means that those groups are substituted by from one to three identical or different groups selected from linear or branched (C₁-C₆)alkyl, linear or branched (C₁-C₆)alkoxy, halogen, hydroxy, linear or branched perhalo-(C₁-C₆)alkyl, nitro, amino (optionally substituted by one or two linear or branched (C₁-C₆)alkyl groups), linear or branched (C₁-C₆)acyl, formyl, carboxy, linear or branched (C₁-C₆)alkoxycarbonyl, aminocarbonyl (optionally substituted on the nitrogen atom by one or two linear or branched (C₁-C₆)alkyl groups), linear or branched (C₁-C₆)acylamino and nitrile.

2. Compounds of formula (I) according to claim 1 wherein n is 1, their enantiomers, diastereoisomers and addition salts thereof with a pharmaceutically acceptable acid or base.

3. Compounds of formula (I) according to claim I wherein n is 0, their enantiomers, diastereoisomers and addition salts thereof with a pharmaceutically acceptable acid or base.

4. Compounds of formula (I) according to claim 1 wherein W represents an SO₂ group, their enantiomers, diastereoisomers and addition salts thereof with a pharmaceutically acceptable acid or base.

5. Compounds of formula (I) according to claim 1 wherein the grouping 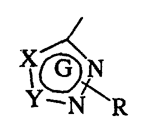 represents a group selected from : 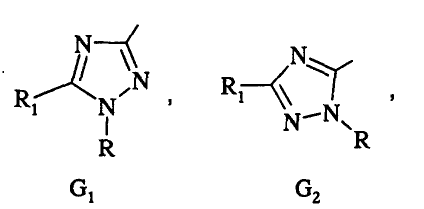 their enantiomers, diastereoisomers and addition salts thereof with a pharmaceutically acceptable acid or base.

6. Compounds of formula (I) according to claim 1 wherein the grouping 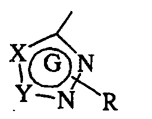 represents a group selected from : 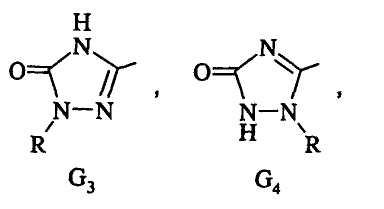 their enantiomers, diastereoisomers and addition salts thereof with a pharmaceutically acceptable acid or base.

7. Compounds of formula (I) according to claim 1 wherein A represents a grouping A₂, their enantiomers, diastereoisomers - and addition salts thereof with a pharmaceutically acceptable acid or base.

8. Compounds of formula (I) according to claim 1 wherein A represents a grouping -A₁-A₂- or -A₂-A₁-, their enantiomers, diastereoisomers and addition salts thereof with a pharmaceutically acceptable acid or base.

9. Compounds of formula (I) according to claim 1 wherein R₁ represents an optionally substituted aryl group, their enantiomers, diastereoisomers and addition salts thereof with a pharmaceutically acceptable acid or base.

10. Compounds of formula (I) according to claim 1 wherein R is selected from a hydrogen atom, an optionally substituted aryl group and an optionally substituted heteroaryl group, their enantiomers, diastereoisomers and addition salts thereof with a pharmaceutically acceptable acid or base.

11. Compounds of formula (I) according to claim 1 wherein Z represents a group selected from alkyl, optionally substituted aryl and optionally substituted heteroaryl, their enantiomers, diastereoisomers and addition salts thereof with a pharmaceutically acceptable acid or base.

12. Compounds of formula (I) according to claim 1 wherein n is 1, W represents an SO₂ group, A represents a group selected from phenylene, pyridinylene and pyrazinylene, R₁ represents an optionally substituted aryl group, R is selected from a hydrogen atom, an optionally substituted aryl group and an optionally substituted heteroaryl group, and Z represents an alkyl, optionally substituted aryl or optionally substituted heteroaryl group, their enantiomers, diastereoisomers and addition salts thereof with a pharmaceutically acceptable acid or base.

13. Compound of formula (I) according to claim 1 which is N'-[4-({5-phenyl-1-[3-(trifluoromethyl)phenyl]-*1H-*1,2,4-triazol -3-yl}amino)benzoyl]benzenesulphonohydrazide.

14. Compound of formula (I) according to claim 1 which is N'-{4-[(5-phenyl-*1H*-1,2,4-triazol-3-yl)amino]benzoyl}benzenesulphonohydrazide.

15. Compound of formula (I) according to claim 1 which is N'-(4-{[5-phenyl-1-(2-pyridyl)-1*H*-1,2,4-triazol-3-yl]amino}benzoyl)benzenesulphonohydrazide.

16. Compound of formula (I) according to claim 1 which is N'-[4-({5-oxo-1-[3-(trifluoromethyl)phenyl]-4,5-dihydro-*1H*-1,2,4-triazol-3-yl}amino)benzoyl}benzenesulphonohydrazide.

17. Compound of formula (I) according to claim 1 which is N'-(4-{[5-oxo-1-(2-pyridyl)-4,5-dihydro-*1H*-1,2,4-triazol-3-yl]amino}benzoyl)benzenesulphonohydrazide.

18. Compound of formula (I) according to claim 1 which is N'-[(6-{[5-oxo-1-(2-pyridinyl)-4,5-dihydro-1*H*-1,2,4-triazol-3-yl]amino}-3-pyridyl)carbonyl]benzenesulphonohydrazide.

19. Process for the preparation of compounds of formula (I) according to claim 1, **characterised in that** there is used as starting material a compound of formula (II) : 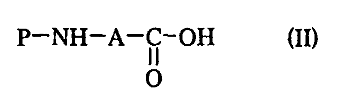 wherein A is as defined for formula (I) and P represents a protecting group for the amine function,
which is reacted in the presence of a coupling agent with a compound of formula (III) :
NH₂-(NH)ₙ-W-Z (III)
wherein n, W and Z are as defined for formula (I),
to yield, after deprotection of the amine function by removal of the P group, a compound of formula (IV) : 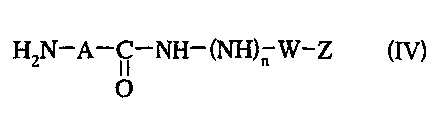 wherein n, A, W and Z are as defined hereinbefore,
which compound (IV) is then condensed in a basic medium with an isothiocyanate of formula (V) : 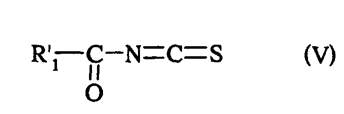 wherein R'₁ has the same meanings as R₁ in formula (I) or represents a linear or branched (C₁-C₆)alkoxy group,
to yield a compound of formula (VI) : 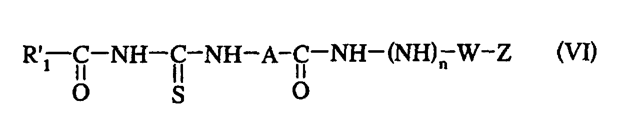 wherein n, R'₁, A, W and Z are as defined hereinbefore,
which compound of formula (VI) is :
- either, when R'₁ represents a linear or branched (C₁-C₆)alkoxy group, condensed in the presence of a coupling agent with a hydrazine of formula R-NH-NH₂, wherein R is as defined for formula (I), to yield a compound of formula (VII/a) : 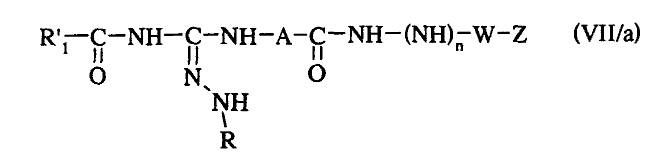
wherein R, A, n, W and Z are as defined hereinbefore, and R'₁ represents a linear or branched (C₁-C₆)alkoxy group,
which compound (VII/a) cyclises, spontaneously or after treatment in an acid medium, depending upon the nature of the R group, to yield a mixture of the two compounds of formulae (I/a) and (I/b) : 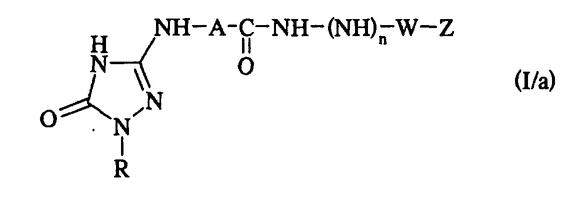 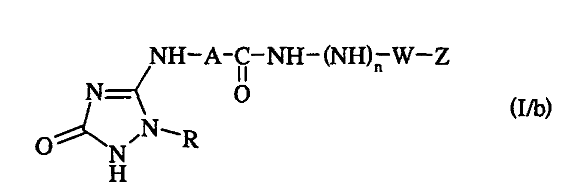 particular cases of the compounds of formula (I) wherein R, A, n, W and Z are as defined hereinbefore,
which compounds (I/a) and (I/b) may be separated according to conventional separation techniques,
- or, when R'₁ represents an R₁ group as defined for formula (I), condensed in the presence of a coupling agent with a hydrazine of formula R-NH-NH₂, wherein R is as defined for formula (I), to yield a compound of formula (VII/b) : 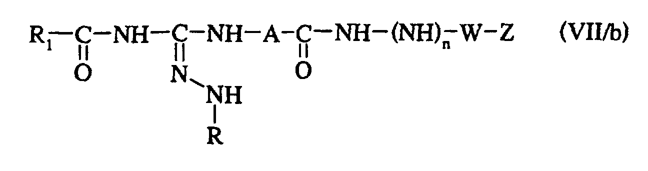
wherein R₁, R, A, n, W and Z are as defined hereinbefore,
which compound (VII/b) is subjected to a cyclisation reaction followed by dehydration, spontaneously or after treatment in an acid medium, depending upon the nature of the R group, to yield a mixture of the two compounds of formulae (I/c) and (I/d) : 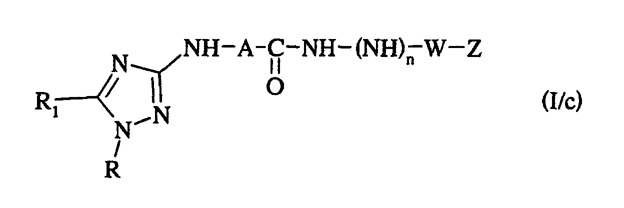 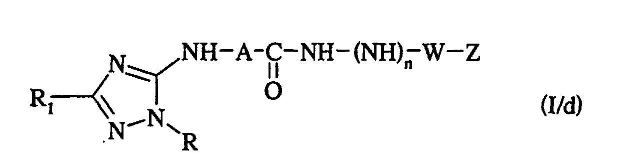 particular cases of the compounds of formula (I) wherein R₁, R, A, n, W and Z are as defined hereinbefore,
which compounds (I/c) and (I/d) may be separated according to conventional separation techniques,
which compounds (I/a), (I/b), (I/c) and (I/d), constituting the totality of the compounds of formula (I),
- are separated, where appropriate, into their enantiomers and/or diastereoisomers according to a conventional separation technique,
- are converted, if desired, into addition salts thereof with a pharmaceutically acceptable acid or base.

20. Pharmaceutical compositions comprising as active ingredient at least one compound according to any one of claims 1 to 18, on its own or in combination with one or more inert, non-toxic, pharmaceutically acceptable excipients or carriers.

21. Pharmaceutical compositions according to claim 20 comprising at least one active ingredient according to any one of claims 1 to 18 for use as Neuropeptide Y receptor ligand in the treatment of pathologies associated with eating behaviour disorders or energy balance disorders, such as diabetes, obesity, bulimia, anorexia nervosa, or in the treatment of arterial hypertension, anxiety, depression, epilepsy, sexual dysfunction and sleep disorders.
